# EUROPEAN PATENT SPECIFICATION

(11) **EP 1 071 764 B1**
(45) Date of publication and mention of the grant of the patent: **12.07.2006**
(21) Application number: 99920590.9
(22) Date of filing: 01.04.1999
(51) Int. Cl.: C12N 15/11, A61K 31/70, C07H 21/04, A61K 47/48

(54) **ANTISENSE OLIGONUCLEOTIDES FOR THE INHIBITION OF INTEGRIN ALPHAV-SUBUNIT EXPRESSION**
ANTISENSE OLIGONUKLEOTIDE ZUR HEMMUNG DER EXPRESSION DER INTEGRIN ALPHAV UNTEREINHEIT
OLIGONUCLEOTIDES ANTISENS DESTINES A INHIBER L'EXPRESSION D'UNE SOUS-UNITE DE L'INTEGRINE ALPHAV

(30) Priority: 17.04.1998 EP 98106989
(43) Date of publication of application: 31.01.2001
(73) Proprietor: Sanofi-Aventis Deutschland GmbH, 65929 Frankfurt am Main (DE)
(72) Inventor: UHLMANN, Eugen, D-61479 Glashütten (DE); PEYMAN, Anuschirwan, D-65779 Kelkheim (DE); TETI, Anna, Maria, I-00040 Ariccia (IT); VILLANOVA, Ida, I-65124 Pescara (IT)
(86) International application number: PCT/EP1999/002286
(87) International publication number: WO 1999/054456

(56) References cited:
- EP-A- 0 653 439
- TOWNSEND P ET AL: "alpha(v) integrin antisense oligodeoxynucleotides induce detachment and apoptosis of osteoclast and breast carcinoma cell." JOURNAL OF BONE AND MINERAL RESEARCH, (AUG 1997) VOL. 12, SUPP. [1], PP. S416, ABSTRACT S252., XP002078998
- NIP J ET AL: "Coordinated expression of the vitronectin receptor and the urokinase-type plasminogen activator receptor in metastatic melanoma cells." JOURNAL OF CLINICAL INVESTIGATION, (1995 MAY) 95 (5) 2096-103., XP002078999 cited in the application
- VILLANOVA J ET AL.: "ANTISENSE OLIGONUCLEOTIDES AGAINST THE ALPHA(V) INTEGRIN SUBUNIT INHIBIT OSTEOCLAST ADHESION AND BONE - RESORPTION" JOURNAL OF BONE AND MINERAL RESEARCH, (AUG 1996) VOL. 11, SUPP. 1, PP. S289, ABSTRACT M431., XP002079000
- MARTIN, P. & SANES, J.: "Integrins mediate adhesion to agrin and modulate agrin signaling" DEVELOPMENT, vol. 124, October 1997 (1997-10), pages 3909-3917, XP002079001 cited in the application
- WADA J ET AL: "CLONING OF MOUSE INTEGRIN ALPHA(V) CDNA AND ROLE OF THE ALPHA(V)-RELATED MATRIX RECEPTORS IN METANEPHRIC DEVELOPMENT" JOURNAL OF CELL BIOLOGY, (MAR 1996) VOL. 132, NO. 6, PP. 1161-1176., XP002079002 cited in the application
- BROOKS P C ET AL: "INTEGRIN ALPHAVBETA3 ANTAGONISTS PROMOTE TUMOR REGRESSION BY INDUCING APOPTOSIS OF ANGIOGENIC BLOOD VESSELS" CELL, vol. 79, 30 December 1994 (1994-12-30), pages 1157-1164, XP002071774 cited in the application
- PANDA, D. ET AL.: "Potential roles of osteopontin and alphaVbeta3 integrin in the development of coronary artery restenosis after angioplasty" PROCEEDINGS OF THE NATIONAL ACADEMY OF SCIENCES OF USA., vol. 94, August 1997 (1997-08), pages 9308-9313, XP002079003 cited in the application
- PEYMAN A ET AL: "MINIMALLY MODIFIED OLIGONUCLEOTIDES-COMBINATION OF END-CAPPING AND PYRIMIDINE-PROTECTION" BIOLOGICAL CHEMISTRY HOPPE-SEYLER, vol. 377, no. 1, January 1996 (1996-01), pages 67-70, XP002041771 cited in the application
- ROZZO C ET AL: "Induction of apoptosis in human neuroblastoma cells by abrogation of integrin -mediated cell adhesion." INTERNATIONAL JOURNAL OF CANCER, (1997 MAR 17) 70 (6) 688-98., XP002079004
- TREASURYWALA, S. & BERENS, M.: "Migration arrest in glioma cells is dependent on the alphaV integrin subunit" GLIA, vol. 24, October 1998 (1998-10), pages 236-243, XP002079005

## Description

The present invention relates to an antisense oligonucleotide or a derivative thereof which has a sequence that corresponds to a part of a nucleic acid which encodes an integrin αᵥ subunit and which has the ability to induce apoptosis, the preparation and the use thereof.

The integrin αᵥβ₃ is the best known vitronectin receptor, however, it is highly promiscous recognizing RGD in a wide array of adhesive proteins, such as fibronectin, fibrinogen, osteopontin, von Willebrand factor, thrombospondin and collagen [Hynes, Cell 69 (1992) 11-12; Horton, Int J. Biochem. Cell Biol. 29 (1997) 721-725].

Despite its promiscous behaviour, integrin αᵥβ₃ is not widely expressed. It is highly expressed on osteoclasts, where it is the dominant integrin. Osteoclasts play a key role in the resorption of bone which leads to osteoporosis. The integrin αᵥβ₃ mediates the adhesion of osteoclasts to bone proteins such as osteopontin or bone sialo-protein, thus stimulating bone resorption. [Chorev, Biochemistry 37 (1995) 367-375]. Inhibition of bone resorption in a rat model of osteoporosis has been reported for the systemic administration of a monoclonal antibody [Crippes, Endocrinology 137 (1996) 918-924.] or by the RGD containing snake venom protein echistatin [Fisher, Endocrinology 132 (1993) 1411-1413] as a proof of concept. This makes the integrin aᵥβ₃ a promising target for the treatment or prevention of osteoporosis.

Integrin αᵥβ₃ is minimally expressed on quiescent blood vessels, but it is significantly upregulated during angiogenesis in vivo [Brooks, Eur. J. Cancer, Part A 32A (1996) 2423-2429; Brooks, DN&P 10 (1997) 456-461]. Angiogenesis plays a role in a variety of pathological states, such as ocular diseases, chronic inflammation, psoriasis, wound healing and cancer. A subsequent blockage of integrin αᵥβ₃ function by antibody or peptide antagonist has been shown to efficiently prevent blood vessel formation in several *in vivo* models. [Stroemblad, Chem. Biol. 3 (1996) 881-885]. Preventing integrin αᵥβ₃ from binding to its ligands leads to apoptosis selectively in proliferating angiogenic vessels [Brooks, Cell 79 (1994) 1157-1164; Ruoslahti, Kidney Int. 51 (1997) 1413-1417, [Meredith, Trends Cell. Biol. 7 (1997) 146-150]. It seems that integrin αᵥβ₃ has a unique function during angiogenesis, namely to provide specific survival signals to facilitate vascular cell proliferation. This function makes integrin αᵥβ₃ an interesting target for the therapy of tumors and the other above mentioned diseases.
Restenosis is additional pathological state that can potentially be avoided by targeting integrin αᵥβ₃. Smooth muscle cells (SMCs) are another site of integrin αᵥβ₃ expression [Hoshiga, Circ. Res. 77 (1995) 1129-1135] and ballon catheder injury induces both osteopontin and integrin αᵥβ₃ mRNA expression [Panda, Proc. Natl. Acad. Sci. USA 94 (1997) 9308-9313]. Integrin αᵥβ₃ seems to promote the survival and migration of SMCs [Leavesley, J. Cell. Biol. 121 (1993) 163-170], [Clyman, Exp. Cell Res. 200 (1992) 272-284]. The blockage of its activity by peptide antagonists reduces neointimal thickening [Matsuno, Circulation 90 (1994) 2203-2206; Choi, J. Vasc. Surg. 19 (1994) 125-134; Yue, Fundam. Clin. Cardiol. 28 (1997) 69-83].

The mAb LM609 against integrin αᵥβ₃ has been used *in vivo* and *in vitro* for the inhibition of angiogenesis in a variety of disease models, such as in a human breast cancer/SCID mouse model [Brooks, J. Clin. Invest. 96 (1995) 1815-1822], in rabbit cormea [Friedlander, Science 270 (1995) 1500-1502], tumor induced angiogenesis on the chick chorioallantoic membrane [Brooks, Cell 79 (1994) 1157-1164]. In summary mAb LM609 leads to a disruption of angiogenesis by inducing apoptosis of angiogenic blood vessels, while no effect is observed on preexisting vessels. This not only prevented the growth of tumors in vivo, but also induced extensive regression in most cases [Brooks, Cell 79 (1994) 1157-1164].

The mAb c7E3 (ReoPro; Centocor/Lilly) which inhibits both integrins αᵥβ₃ and α_{||b}β₃ recently demonstrated in completed Phase III trials effective reduction in post angioplasty occlusive events, as well as late events associated with restenosis [Topol, Am. J. Cardiol. 75 (1995) 27B-33B].

17E6, an antibody against the integrin αᵥ subunit, recting with the integrins αᵥβ₃, αᵥβ₅, and αᵥβ₁, strongly inhibited tumor development in nude mouse tumor models [Mitjans, J. Cell. Sci. 108 (1995) 2825-2838].

The cyclic peptide [cyclo-RGDfV] which selectively binds to integrin αᵥβ₃ (IC₅₀ = 49 nM) (lower case letters indicate D-amino acids) [Pfaff, J. Biol. Chem. 269 (1994) 20233-20238; Wermuth, J. Am. Chem. Soc. 119 (1997) 1328-1335] has also been successfully used by Brooks et al. [Brooks, Cell 79 (1994) 1157-1164] to inhibit tumor induced angiogenesis on the chick chorioallantoic membrane.

Another possibility of inhibition of function of integrin αᵥβ₃ is the use of antisense oligonucleotides [E. Uhlmann and A. Peyman, Chemical Reviews 90, 543 (1990); S. Agrawal, TIBTECH 1996, 376]. The synthesis of integrin αᵥ subunit in melanoma cells was suppressed by a 18-mer integrin αᵥ subunit all-phosphorothioate antisense oligonucleotide [Nip, J. Clin. Invest. 95 (1995) 2096-2103]. Inhibition of mouse integrin αᵥ subunit using a mouse-specific 21-mer αᵥ antisense oligonucleotide [Martin, P. T., & Sanes, J. R. (1997) Development 124, 3909-3917] and a mouse-specific 43-mer αᵥ antisense oligonucleotide [Wada, J., Kumar, A., Liu, Z., Ruoslahti, E., Reichardt, L., Marvaldi, J., & Kanwar, Y. S. (1996) J. Cell Biol. 132, 1161-76] has also been described. No induction of apoptosis was obesrved on treatment with any of the three described oligonucleotides. In all three cases, the antisense oligonucleotides were used as all-phosphorothiates.
Nip et al described an antisense oligonucleotide AS-3, which is directed against nucleotides 41-58 of the human vitronectin receptor αᵥ subunit (HSVTNR = human integrin αᵥβ₃; AC M14648). This region encompasses the translational start site of HSVNTR.

The abstract "The αᵥ integrin prevents apoptosis in Bone metastatic Breast Cancer Cells (Townsed et al.; diclosed on the ASBMR Meeting in 1998) summarizes effects of integran αᵥ antisense oligonucleotides, but without disclosing a particular sequence and structure of the oligonucleotide.

The general concept of the invention is to provide an oligonucleotide or a derivative thereof which
a) has a sequence that corresponds to a part of a nucleic acid which encodes an integrin αᵥ subunit (= integrin αᵥ) and
b) has the ability to induce apoptosis.

The present invention provides an antisense oligonucleotide or a derivative thereof which has or comprises one of the sequences according to one of the SEQ ID NO. 4, SEQ ID NO. 5, SEQ ID NO. 6, SEQ ID NO. 7, SEQ ID NO. 8 or SEQ ID NO. 12, and which
a) has a sequence that corresponds to a part of a nucleic acid which encodes infiegrin αᵥ and
b) induces apoptosis in a cell that contains integrin αᵥ mRNA, when brought into that cell.

The antisense oligonucleotide or a derivative thereof modulates the expression of at least one protein which is involved in a signal transduction pathway that induces apoptosis when brought into contact with a cell.

The antisense oligonucleotide or a derivative thereof induces for example an increase in p21 expression, a reduction in bcl-2 expression and/or when brought into a cell induces an inhibition of cell adhesion; for example when brought into contact with an osteoclast cell the antisense oligonucleotide or a derivative thereof induces an inhibition of bone resorption; and/or induces a translocation of p53 from the cytosol to the nucleus of that cell.

Further, the present invention provides an antisense oligonucleotide or a derivative thereof, which has or comprises one of the sequences according to one of the SEQ ID NO. 4, SEQ ID NO. 5. SEQ ID NO. 6, SEQ ID NO. 7. SEQ ID NO. 8 or SEQ ID NO. 12, and which
a) has a sequence that corresponds to a part of a nucleic acid which encodes an integrin αᵥ and
b) which when brought into a cell inhibits the adhesion of that cell to particular substrates.

Said antisense oligonudeotide or a derivative thereof may inhibit the adhesion of a cell to a substrate that comprises at least one protein that belongs to the group of extracellular matrix proteins, serum proteins, bone sections, vitronectin, fibrinogen and/or fibronectin.

The present invention further relates to a process for the preparation of said oligonucleotide or a derivative thereof wherein suitably protected monomers are condensed on a solid support.

Further, the invention comprises a method for inhibiting the expression of integrin αᵥ, wherein an antisense oligonucleotide or a derivative thereof with one of the sequences SEQ ID NO. 4 to SEQ ID NO. 8 or SEQ ID NO. 12 is made and hybridized to integrin αᵥ mRNA in vitro.

The invention in addition relates to a method for inhibiting the adhesion of a cell cultured in vitro to a particular substrate, wherein said antisense oligonucleotide or a derivative thereof, which has a sequence that corresponds to a part of a nucleic acid that encodes integrin αᵥ is made and brought into that cell, whereupon the antisense oligonucleotide or derivative thereof hybridizes to integrin αᵥ mRNA and thereby inhibits the expression of integrin αᵥ to a certain extend.

Further, the invention comprises a method of modulating the expression or activity of a protein which is involved in at least one of the signal transduction pathways for inducing apoptosis, wherein said antisense oligonucleotide or a derivative thereof, which has a sequence that corresponds to a part of a nucleic acid that encodes for integrin αᵥ is made and brought into a cell cultured in vitro, whereupon the antisense oligonucleotide or the derivative thereof hybridizes to integrin αᵥ mRNA and thereby inhibits the expression of integrin αᵥ to certain extend.

The invention also concerns the use of said antisense oligonucleotide or a derivative thereof for the preparation of a pharmaceutical composition for treating diseases which are associated with the expression or a increased expression of integrin αᵥ.

Another aspect of the invention relates to a method for preparing a pharmaceutical composition, which comprises mixing of at least one antisense oligonucleotide according to the invention or a derivative thereof with a physiologically acceptable excipients and if appropriate suitable additives and/or auxiliaries.

The invention also relates to a pharmaceutical composition which comprises at least one of the described oligonucleotides or a derivative thereof and if appropriate one or more physiologically acceptable excipients and suitable additives and/or auxiliaries; in particular, it relates to the pharmaceutical composition according to the invention for the treatment and/or prevention of diseases which are associated with abnormal cell proliferation, cell migration, cell differentiation, angiogenesis, retinal neurite outgrowth, bone resorption, phagocytosis, immune response, signal transduction and the metastasis of neoplastic cells or to the pharmaceutical composition according to the invention, for the treatment and prevention of cancer and metastasis of cancer, the treatment and prevention of osteoporosis, the treatment of ocular diseases, chronic inflammation, psoriasis, restenosis and the support of wound healing.

In another aspect the invention relates to a diagnostic reagent and/or a test kit and further to a method for identifying cells, which express or overexpress integrin αᵥ, wherein said oligonucleotide or a derivative thereof is synthesized and brought into contact with a cell or a probe of a cell, and to determine if the antisense oligonucleotide or derivative thereof has hybridized to integrin αᵥ mRNA,

Such test kit or diagnostic reagent might be suitable for identifying cells which express or overexpress integrin α_{v;} the test kit or diagnostic reagent comprises
a) said oligonucleotide or a derivative thereof that has a sequence that corresponds to a part of a nucleic acid which encodes for integrin αᵥ and
b) a reagent for detecting if the oligonucleotide or derivative thereof has hybridized to integrin αᵥ mRNA.

The part of the nucleic acid to which the oligonucleotide (hereinafter "ON") corresponds has a length of 5 to 100 nucleotides or more, preferably of 8 to 26 nucleotides, most preferred is a length of 10 to 20 nucleotides. Therefore an oligonucleotide of the invention has a length of 5 to 100 nucleotides or more, preferably of 8 to 26 nucleotides, most preferred is a length of 10 to 20 nucleotides. In special embodiments of the invention the oligonucleotide has a length of 18, 16, 14 or 12 nucleotides.

The oligonucleotide has a sequence that corresponds to a part of a nucleic acid which encodes an integrin αᵥ subunit. "Corresponds" to means that the order of bases of the oligonucleotide allows the oligonucleotide to hybridize to that part of the nucleic acid to which the oligonucleotide sequence corresponds. With respect to the nucleic acid the order of nudeobases within the oligonucleotide sequence might be the same ("sense oligonucleotide") or the opposite ("antisense oligonucleotide").

Furthermore, the oligonucleotide might have one or more mismatches in comparison to the nucleic acid sequence.

The oligonucleotide might hybridize to mRNA, double and single stranded DNA or cDNA respectively, The oligonucleotide might be an antisense oligonucleotide, a triple helix forming oligonucleotide, a ribozyme or a aptamer. In a preferred embodiment of the invention the oligonucleotide is an antisense oligonucleotide.

The nucleic acid sequence which encodes integrin αᵥ subunit might be any sequence which encodes the 125 kDa fragment or the 25 kDa fragment of the integrin αᵥ subunit or a part thereof. The nucleic acid might be a cDNA, mRNA or a gene or a part thereof. The source of the nucleic acid sequence might be any animal, preferably a mammalian animal, most preferably a human. In a preferred embodiment of the invention, the oligonucleotide sequence is deduced from or corresponds to respectively a part of the human integrin αᵥ subunit cDNA.

The sequence of the human integrin αᵥ subunit cDNA is available from gene-databases such as e.g. EMBL or NCBI. Human integrin αᵥ subunit sequences can for example be obtained with the accession numbers M14648, J02826 and M18365. A part of the human integrin αᵥ subunit cDNA is available from Suzuki et al. (1986) Proc. Natl. Acad. Sci. USA 83, p. 8616. In a preferred embodiment of the invention the oligonucleotide corresponds to a part of SEQ ID NO. 1 (Table 1).

Within SEQ ID NO. 1 two preferred regions exist: a) core region 1 (SEQ ID NO. 2): nucleotides 37-60 and core region 2 (SEQ ID: NO. 3): nucleotides 122-147 of the HSVTNR gene shown in SEQ ID NO. 1 (core regions are underlined).
- SEQ ID NO. 2:: 5'- CGGC GATGGCTTTT CCGCCGCGGC -3'
- SEQ ID NO. 3:: 5'- GTGCCGCGC CTTCAACCTA GACGTGG -3'

Examples for sequences for an oligonucleotide are:
- SEQ ID NO. 4:: 3'- GAAGCCGCTACCGAAAAGGC -5'
- SEQ ID NO. 5:: 3'- CGCGTGAAGCCGCTACCG -5'
- SEQ ID NO. 6:: 3'- GCTACCGAAAAGGCGGCG -5'
- SEQ ID NO. 7:: 3'- GCTGCCGAGAGAGCAACG -5'
- SEQ ID NO. 8:: 3'- GCGGAAGTTGGATCTGC -5'
- SEQ ID NO. 12:: 3'- GCGGAAGTTGGACCTGC -5'

In a special embodiment of the invention the oligonucleotide has the sequence SEQ ID NO. 6 or a part thereof or the oligonucleotide sequence is deduced thereof (e.g. it has one or more mismatches). In another special embodiment of the invention, the oligonucleotide comprises the sequence SEQ ID NO. 6.

The cDNA of mouse integrin αᵥ subunit is e,g. disclosed in Wada et al. (1996) J. Cell Biol. 132, p. 1165. An example for an oligonucleotide sequence is SEQ ID NO. 9.
- SEQ ID NO. 9:: 3'- GCTAGCGACGAGGGCCCG -5'

The invention also relates to derivatives of the oligonucleotides, for example their salts, in particular their physiologically tolerated salts. Salts and physiologically tolerated salts are described in Remingtons Pharmaceuticals Science (1985) Mack publishing Company, Easton, PA (page 1418). Derivatives also relate to modified oligonucleotides which have one or more modifications (e.g. at particular nucleoside positions and/or at particular internucleoside bridges, oligonucleotide analogues (e.g. Polyamid Nucleic Acids (PNAs), Phosphoric acid monoester nucleic acids (PHONAs = PMENAs), oligonucleotid chimeras (e.g. consisting of a DNA- and a PNA-part or consisting of a DNA- and a PHONA-part).

Subject of the invention is an antisense oligonucleotide or a derivative thereof which has or comprises one of the sequences according to one of the SEQ ID NO. 4, SEQ ID NO. 5, SEQ ID NO. 6, SEQ ID NO. 7. SEQ ID NO. 8 or SEQ ID NO. 12, and which
a) has a sequence that corresponds to a part of a nucleic acid which encodes integrin αᵥ and
b) induces apoptosis in a cell that contains integrin αᵥ mRNA, when brought into that cell,

in particular an antisense oligonucleotide directed against the human integrin αᵥ subunit mRNA (HSVTNR mRNA), which is modified to make it resistant against nucleases with the provisio that this oligonucleotide is not uniformly modified with phosphorothioate internucleotide bridges (all-phosphorothiates) and that it induces apoptosis of the correspondingly treated cells.

An oligonucleotide can, for example, be composed completely of the "natural nucleotides" (comprising the "naturanucleoside bases") deoxyadenosine phosphate, deoxyguanosine phosphate, deoxyinosine phosphate, deoxycytidine phosphate, uridine phosphate and thymidine phosphate. In other embodiments of the invention, an oligonucleotide can, where appropriate, contain one or more modifications, for example chemical modifications. An oligonucleotide can exhibit several identical and/or different modifications. The coording modified oligonucleotide encompasses oligonucleotide analogues and oligonucleotid chimeras.

Examples of chemical modifications are known to the skilled person and are described, for example, in E. Uhlmann and A. Peyman, Chemical Reviews 90 (1990) 543 and "Protocols for Oligonucleotides and Analogs" Synthesis and Properties & Synthesis and Analytical Techniques, S. Agrawal, Ed, Humana Press, Totowa, USA 1993 and S.T. Crooke, F. Bennet, Ann. Rev. Pharmacol.Toxicol. 36 (1996) 107-129.

The invention relates to an antisense oligonucleotide which has or comprises one of the sequences according to one of the SEQ ID NO.4, SEQ ID NO.5, SEQ ID NO. 6, SEQ ID NO.7, SEQ ID NO.8 or SEQ ID NO. 12, and which
a) has a sequence that corresponds to a part of a nucleic acid which encodes integrin αᵥ and
b) induces apoptosis in a cell that contains integrin αᵥ mRNA, when brought into that cell,
and which comprises one or more modifications and wherein each modification is independently selected from
a) the replacement of a phosphoric diester bridge located at the 3'- and/or the 5'- end of a nudeoside by a modified phosphodiester bridge,
b) the replacement of a phosphoric diester bridge located at the 3'- and/or the 5"- end of a nucleoside by a dephospho bridge,
c) the replacement of a sugar phosphate molecule from the sugar phosphate backbone by another unit,
d) the replacement of a β-D-2'-deoxyribose by a modified sugar radical,
e) the replacement of a natural nucleoside base by a modified nucleoside base,
f) the conjugation of the oligonucleotide to a molecule which influences the properties of the oligonucleotide and
g) the introduction of a 3'-3' and/or a 5'-5' inversion at the 3' and/or the 5' end of the oligonucleotide.

More detailed examples for the chemical modification of an oligonucleotide are
a) the replacement of one or more phosphoric diester bridge by modified phosphodiester bridges, for example with phosphorothioate, phosphorodithioate, NR¹R^{1'}-phosphoramidate, boranophosphate, phosphate-(C₁-C₂₁)-O-alkyl ester, phosphate-[(C₆-C₁₂)aryl-((C₁-C₂₁)-O-alkyl]ester, (C₁-C₈)alkyl-phosphonate and/or (C₆-C₁₂)-arylphosphonate bridges,
   where R¹ and R^{1'} are, independently of each other, hydrogen, (C₁-C₁₈)-alkyl, (C₆₋C₂₀)-aryl, (C₆-C₁₄)-aryl-(C₁-C₈)-alkyl, preferably hydrogen, (C₁-C₈)-alkyl and/or methoxyethyl, preferably hydrogen in particular, (C₁-C₄)-alkyl and/or methoxyethyl, or
   R¹ and R^{1'} form, together with the nitrogen atom carrying them, a 5-6-membered heterocyclic ring which can additionally contain a further heteroatom from the group O, S and N;
b) the replacement of one or more 3'- and/or 5'-phosphoric diester bridges (phosphodiester bridges) with "dephospho" bridges (described, for example, in Uhlmann, E. and Peyman, A. in "Methods in Molecular Biology", Vol. 20, "Protocols for Oligonucleotides and Analogs", S. Agrawal, Ed., Humana Press, Totowa 1993, Chapter 16, 355ff), for example with formacetal, 3'-thioformacetal, methylhydroxylamine, oxime, methylenedimethylhydrazo, dimethylenesulfone and/or silyl groups;
c) the replacement of one or more sugar phosphate units from the sugar phosphate backbone by another unit, for example with a unit suitable to built up a "morpholinoderivative" oligomer (described, for example, in E.P. Stirchak et al., Nucleic Acids Res. 17 (1989) 6129) and/or a polyamide nucleic acid ("PNA") (described, for example, in P.E. Nielsen et al., Bioconj. Chem. 5 (1994) 3), e.g. 2-aminoethylglycine and/or a phosphoric acid monoester nucleic acid (phosphomonoacidic ester nucleic acid; "PHONA") (described, for example, in Peyman et al., Angew. Chem. Int. Ed. Engl. 35 (1996) 2632-2638 and in EP 0 738 898 A2);
d) the replacement of one or more β-D-2'-deoxyribose units with a modified sugar radical, for example with β-D-ribose, α-D-2'-deoxyribose, L-2'-deoxyribose, 2'-F-2'-deoxyribose, 2'-O-(C₁-C₆)alkyl-ribose, 2'-O-(C₂-C₆)alkenyl-ribose, 2'-[O-(C₁₋C₆)alkyl-O-(C₁-C₆)alkyl]-ribose, 2'-NH₂-2'-deoxyribose, β-D-xylofuranose, α-arabinofuranose, 2,4-dideoxy-β-D-erythro-hexo-pyranose, and carbocyclic (described, for example, in Froehler, J. Am. Chem. Soc. 114 (1992) 8320) and/or open-chain sugar analogs (described, for example, in Vandendriessche et al., Tetrahedron 49 (1993) 7223) and/or bicyclosugar analogs (described, for example, in M. Tarkov et al., Helv. Chim. Acta 76 (1993) 481);
e) the modification or replacement of one or more natural nucleoside bases with modified nucleosid bases, for example with 5-(hydroxymethyl)uracil, 5-aminouracil, pseudouracil, dihydrouracil, 5-(C₁-C₆)-alkyl-uracil, 5-(C₂-C₆)-alkenyl-uracil, 5-(C₂-C₆)-alkynyl-uracil, 5-(C₁-C₆)-alkyl-cytosine, 5-(C₂-C₅)-alkenyl-cytosine, 5-(C₂-C₆)-alkynyl-cytosine, 5-fluorouracil, 5-fluorocytosine, 5-chlorouracil, 5-chlorocytosine, 5-bromouracil, 5-bromocytosine or 7-deaza-7-substituted purines.
f) the conjugation of the oligonucleotide with one or more molecules which (favorably) influence the properties (for example cell penetration, nuclease stability, affinity for the target nucleic acid e.g. the HSVTNR target sequence and/or improve pharmacokinetics) of the oligonucleotide (e.g. the properties of an antisense oligonucleotide and/or of a triple helix-forming oligonucleotide); such molecule can attack the target nucleic acid sequence, while binding and/or crosslinking, when the modified oligonucleotide hybridizes with this target sequence; examples are conjugates with polylysine, with intercalating agents such as pyrene, acridine, phenazine or phenanthridine, with fluorescent compounds such as fluorescein, with crosslinking agents such as psoralen or azidoproflavin, with lipophilic molecules such as (C₁₂-C₂₀)-alkyl, with lipids such as 1,2-dihexadecyl-rac-glycerol, with steroids such as cholesterol or testosterone, with vitamins such as vitamin E, with poly- or oligoethylene glycol, with (C₁₂-C₁₈)-alkyl phosphate diesters and/or with O-CH₂-CH(OH)-O-(C₁₂-C₁₈)-alkyl; these molecules can be conjugated at the 5' end and/or the 3' end and/or within the sequence, e.g. to a (modified) nucleoside base; a special embodiment of the chemical modification relates to the conjugation of the oligonucleotide a) with lipophilic molecules, for example (C₁₂-C₂₀)-alkyl, b) with steroids such as cholesterol and/or testosterone, c) with poly- and/or oligoethylene glycol, d) with vitamin E, e) with intercalating agents such as pyrene, f) with (C₁₄₋C₁₈)-alkyl phosphate diesters and/or g) with O-CH₂-CH(OH)-O-(C₁₂-C₁₈)-alkyl; processes for preparing an oligonucleotide conjugate are known to the skilled person and are described, for example, in Uhlmann, E. & Peyman, A., Chem. Rev. 90 (1990) 543 and/or M. Manoharan in "Antisense Research and Applications", Crooke and Lebleu, Eds., CRC Press, Boca Raton, 1993, Chapter 17, p. 303ff, and EP-A 0 552 766;
g) in other, special embodiment of the invention, the oligonucleotide can exhibit 3'-3' and/or 5'-5' inversions at the 3' and/or the 5' end; this type of chemical modification is known to the skilled person and is described, for example, in M. Koga et al, J. Org. Chem. 56 (1991) 3757.

In a special embodiment of the invention the oligonucleotide corresponds to a part of the nucleic acid sequence near the translational start site (SEQ ID NO. 1, nucleotides 1 to 60) (Tables 1 and 2) which is known to be in many instances an efficient region for antisense oligonucleotides. A number of oligonucleotides which have a sequence that corresponds to a part of SEQ ID NO. 1 and which were additionally modified were synthesiszed and characterized. Surprisingly, it was found that an antisense oligonucleotide against nucleotides 50-67 of HSVTNR (numbers refer to SEQ ID NO. 1, Tables 1 and 2) which is named ON 5543 is more effective for the inhibition of HSVTNR expression than other oligonucleotides which are directed against (or correspond to respectively) nucleotides 44-63 (ON 5541), 41-58 (AS-3 from Nip et al.) and 39-56 (ON 5542).

The sequence of ON 5543 corresponds to nucleotides 50-67, ON 5541 corresponds to nucleotides 44-63, AS-3 (Nip et al) corresponds to nucleotides 41-48 and ON 5542 corresponds to nucleotides 39-56.

The oligonucleotides ON 5543, ON 5541 , ON 5542 and AS-3 (Nip et al) are modified. Oligonucleotide AS-3 is an all phosphothioate, all phosphodiester bridges are replaced by phosphothioat bridges. Within the other oligonucleotides only particular positions are modified: The localisation of modification within the sequence is shown (the type of modification: replacement of phosphodiester bridges by thiophosphate bridges):
SEQ ID NO. 4: (ON 5541)
   3'-G*A*AGC*C*GC*TAC*C*GAAAAG*G*C -5'
SEQ ID NO. 5: (ON 5542) antisense
   3'-C*G*C*GT*GAAG*C*CGC*TA*C*C*G -5'
SEQ ID NO. 6: (ON 5543) antisense
   3'-G*C*T*AC*CGAAAAGG*CGG*C*G -5'
* is a phosphorothioate residue.

The invention relates also to oligonucleotides which have modifications at the same positions, but another type of modification.

Other oligonucleotides are used as control oligonucleotides to determine sequence specificity of the above oligonucleotides. For example the oligonucleotides SEQ ID. NO. 10, 11 and 7 are used (control oligonucleotides):
SEQ ID NO. 10: ON 5043 (inverted control of ON 5543)
   5'-G*C*T*AC*CGAAAAGG*CGG*C*G-3'
SEQ ID NO. 11: ON 5044 (sense of ON 5543)
   5'-C*G*ATGGC*TT*TT*CCGCC*G*C-3'
SEQ ID NO. 7: ON 5045 (mismatch of ON 5543)
   3'-G*C*T*GC*CGAGAGAG*CAA*C*G-5'
* is a phosphorothioate residue., mismatches in sequence SEQ ID NO. 7 are underlined.

The observed inhibition with the antisense oligonucleotides is specific for the target protein (target protein is integrin αᵥ subunit) or the target sequence (a nucleic acid which encodes the integrin αᵥ subunit) respectively, since only the αᵥ protein levels were reduced, while other protein levels remain unchanged; as a reference e.g. b₃ and actin protein levels can be determined: they remained unchanged. Thus, theses oligonucleotides specifically inhibit the expression of integrin αᵥ subunit. Furthermore, also shortened versions of ON 5543 can block or inhibit respectively the expression of the human vitronectin receptor αᵥ subunit.

In addition, the partially phosphorothioated oligonucleotide ON 5543 showed a higher specificity than the all-phosphorothioates used in previous studies (Nip et al., Martin et al.; Wada et al.).

A second region on the HSVTNR RNA was identified wihtin the coding region, nucleotides against which effective antisense oligonucleotides could be identified. For example, ON 5959 covering nucleotides 128-145 (corresponding to nucleotides 128-145) could efficiently inhibit integrin αᵥ protein synthesis. This region shows high sequence homology between different species, such as human, chicken and mouse.

A Comparison of human, chicken and mouse sequences and the respective antisense oligonucleotide(s) (sequences) are shown:

| | | | |
|---|---|---|---|
| human | 128 | 5'- CGCCTTCAACCTAGACG -3' | 145 |
| gallus | | 5'- CGCCTTCAACCTGGACG -3' | |
| mouse | | 5'- CGCCTTCAACCTGGACG -3' | |

The corresponding antisense sequence:
SEQ ID NO. 12:
   3'- GCGGAAGTTGGACCTGC -5'
SEQ ID NO. 12: ON 5473 ("consensus-1" antisense)
   3'- G*C*G G A A G*T*T G G A C*C*T G*C -5'
SEQ ID NO. 13: ON 5474 (inverted control)
   5'- G*C*G G A A G*T*T G G A C*C*T G*C 3'
SEQ ID NO. 14: ON 5475 (sense)
   5'- C*G C*C T*T*C A A C*C*T G G A*C*G 3'
SEQ ID NO. 8: ON 5959 ("consensus-2" antisense)
   3'- G*C*G G A A G*T*T G G A T*C*T G*C -5'
underlined: 5-propinyl pyrimidines, * phosphothioate bridges

The oligonucleotide "consensus-1" has one mismatch against human, but is perfect against mouse and chicken.
The oligonucleotide "consensus-2" is perfect against human, but has one G-T-mismatch against mouse and chicken.

In a particular embodiment of the invention, an oligonucleotide is prepared by only replacing some of the phosphodiester bridges with phosphorothioate bridges. In particular, the invention comprises oligonucleotides which are only modified to a minimal extent. The principle of minimally modified oligonucleotides is described in A. Peyman, E. Uhlmann, Biol. Chem. Hoppe-Seyler, 377 (1996) 67-70 and EP 0 653 439. In this case, 1-5, more preferably 1-3 terminal nucleotide units at the 5' end/or and at the 3' end are protected, for example the phosphodiester intonucleoside bridges located at the 3' and/or the 5' end of the corresponding nucleosides are for example replaced by phosphorothioate internucleoside bridges. In addition, preferably at least one internal pyrimidine nucleoside is modified. Preferably the 3' and/or the 5' internucleoside bridge(s) of an internal pyrimidine nucleoside is/are modified/replaced, e.g. by phosphorothiate bridges. Minimally modified oligonucleotides exhibit particularly advantageous properties; for example they exhibit a particularly high degree of nuclease stability in association with minimal modification. They also have a significantly reduced propensity for non-antisense effects which are often associated with the use of all-phosphorothioate oligonucleotides (Stein anal. Krieg (1994) Antisense Res. Dev. 4, 67). Partially modified oligonucleotides also show a higher binding affinity than all-phosphorothioates.

In a special embodiment of the invention at the 3' and/or the 5'end of the oligonucleotide 1-5 nucleotides are modified. In another special embodiment of the invention at least one non-terminal pyrimidine nucleoside and/or a phosphodiester bridge located at the 3' and/or the 5' end of that pyrimidine nucleoside is modified.

Special embodiments of the invention include a minimally modified oligonucleotide. For example, a minimally modified oligonucleotide can exhibit the following phosphorothioate patterns:
- SEQ ID NO. 6:: 5'-G*C*GGC*GGAAAAGC*CA*T*C*G-3'
- SEQ ID NO. 8:: 5'-C*GT*C*TAGGT*T*GAAGG*C*G -3'
("*" denotes phosphorothioate bridge)

These minimally modified oligonucleotides can, for example, also include other types of modifications, e.g. of the nucleoside bases, e.g. be substituted by 5-propynylpyrimidines, as well:
- SEQ ID NO. 8:: 5'-C*GT*C*TAGGT*T*GAAGG*C*G -3'
(C : 5-Propinylcytosin, T : 5-Propinyluracil; "*"phosphorothioate bridge)

A further prefered embodiment is constituted by chimeric oligonucleotides composed of DNA and 2'-O-methyl-RNA, for example:
- SEQ ID NO. 6:: 5'-G*C*GGC*GGAAAAGC*CA*T*C*G-3'
- SEQ ID NO. 8:: 5'-C*GT*C*TAGGT*T*GAAGG*C*G -3
("*" denotes phosphorothioate bridges, 2'-O-methyl modified nucleotides 2'-O-methyl-ribonucleosides are underlined)

A further prefered embodiment is constituted by chimeric oligonucleotides composed of DNA and PNA, for example:
- SEQ ID NO. 6:: 5'-G*C*GGC*Ggaaaagccatcg-3'
- SEQ ID NO. 8:: 5'-C*GT*C*TAggttgaaggcg-3'
("*" denotes phosphorothioate bridges, the sequence of the PNA moiety is indicated by small letters)
- SEQ ID NO. 6:: 5'-G*C*GGC*Ggaaaagccatcg-3'
- SEQ ID NO. 8:: 5'-C*GT*C*TAggttgaaggcg-3'
("*" denotes phosphorothioate bridges, the sequence of the PNA part is indicated by small letters, the 2'-O-methyl-modified nucleotides (2'-o-methylribonucleoside unit) are underlined)

A further preferred embodiment is constituted by antisense oligonucleotides having hexadecyl (C16) residues at the 3' or 5' end, for example:
- SEQ ID NO. 6:: 5'-C16-G*C*GGC*GGAAAAGC*CA*T*C*G-3'

The oligonucleotides of the invention display a characteristic funtional activity: they efficiently inhibit αᵥ (= integrin αᵥ subunit) protein synthesis; this can for example be demonstrated when the amount of protein is determined relative to probes with the control oligodesoxynucleotides (ON s). Furthermore, treatment of osteoclasts with the oligonucleotides of the invention induced a dose-dependent, substrate-specific reduction of osteoclast adhesion and inhibited bone resorption with a low IC₅₀ (2x10¹⁰µM). The oligonucleotides of the invention caused morphological changes consistent with cell retraction, and induced apoptosis in the treated cells as observed by DNA staining with bis-benzimide, and confirmed by decoration of early-stage fragmented DNA by TUNEL. Surprisingly, the oligonucleotides of the invention stimulated the expression of the cyclin/cyclin-dependent kinase complex inhibitor p₂₁^{WAF1/CIP1}, and inhibited that of the cell survival gene, bcl-2. In contrast, the expression of the cell death promoting gene bax remained unchanged. This led to a reduction of the bcl-2/bax ratio which is known to underly the intracellular signal to apoptosis.

The oligonucleotides of the invention were also active in various cancer cells lines. Thus, Oligonucleotide ON 5543 inhibited cell attachment of breast carcinoma MDA-MB231 cells whereas the corresponding sense and mismatch control oligonucleotides had no effect. The cell adhesion was blocked in a dose-dependent manner at 10 nM to 1 µM antisense oligonucleotide concentrations. Surprisingly, induction of apoptosis was also observed for the cancer cells treated with the oligonucleotides of the invention which makes them useful for tumor therapy.

Surprisingly different downsteam signaling mechanisms are effected upon treatment with integrin αᵥ submit specific antisense oligonucleotides: the mechanism that is affected to induce apoptosis depends on the cell-typ (cell type-specific functional activity of the ONs).

Therefore, the present invention relates to an oligonucleotide which is characterized by a specific and unexpected function (functional acitivity): When the oligonucleotide is brought into contact with a cell, it modulates the expression of at least one protein which is involved in apoptosis: In a first aspect, the invention relates to an oligonucleotide which induces an increase in p21 expression, in particular in p₂₁^{WAF1/CIP1} expression. In a second embodiment, the invention relates to an oligonucleotide, which induces a reduction in bcl-2 expression. In a third embodiment, the invention relates to an oligonucleotide, which did not affect the expression of bax. In a another embodiment of the invention, the oligonucleotide induces a reduction in bcl-2 expression, but has no effect on the expression of bax. In this embodiment of the invention the oligonucleotide causes a reduction of the bcl-2/bax ratio, the oligonucleotide causes an intracellular signal to apoptosis.

The oligonucleotide is characterized by further specific functions: In another embodiment of the invention the oligonucleotide induces an inhibition of cell adhesion, preferably this effect is dose-dependent, in a prefered embodiment the oligonucleotide inhibits anchorage-dependent growth. In another embodiment of the invention the oligonucleotide induces an inhibition of bone resorption; preferably, this effect is dose-dependent.

The invention further relates to the process for the preparation of an oligonucleotide according to the invention. The process for preparation comprises the chemical synthesis of the oligonucleotide. Preferably the chemical synthesis is a standard methode used for the sythesis of oligonucleotides, e.g. the phoshoamidite methode described in example 1.

The invention further relates to methods of using the oligonucleotide as antisense oligonucleotide, as triple-helix forming oligonucleotide, as adaptamer or as ribozyme. Further, the invention relates to methods, wherein the oligonucleotide is used to inhibiting the expression of integrin αᵥ subunit, for modulating the expression of at least one protein which is involved in apoptosis, for inducing apoptosis in a cell, for inhibiting cell adhesion and for inhibiting bone resorption, for inhibiting angiogenesis and vascularisation, e.g. by inducing apoptosis of angiogenetic blood vessels, in particular if this vascularisation is associated with the growth of tumors and with metastasis of tumors. Therefore, the invention relates to methods of using of the oligonucleotides for the treatment of tumors and for preventing metastasis.

The invention further relates to a methode of inhibiting the expression of integrin αᵥ subunit and/or modulating the expression of a protein which is involved in apoptosis and/or inducing apoptosis and/or inhibiting cell adhesion and/or inhibiting bone resporption, wherein an oligonucleotide of the invention is brought into contact with a cell.

The invention furthermore relates to the use of the oligonucleotides for modulating and also totally or partially inhibiting the expression of the αᵥ protein and/or mutants thereof, for example for totally or partially inhibiting translation. Therefore, the invention relates to a methode, wherein the oligonucleotide is brought into contact with a cell and a methode for introducing the oligonucleotide in a cell. The invention further relates to a methode for hybridizing the oligonucleotide with a target nucleic acid and to a methode of inhibiting the expression of the integrin αᵥ target nucleic acid.

The invention furthermore relates to the use of the oligonucleotides as pharmaceuticals and to the use of the oligonucleotides for preparing pharmaceuticals. In particular, the oligonucleotides can be used in pharmaceuticals which are employed for preventing and/or treating diseases which are associated with the expression or an overexpression (increased expression) of the integrin αᵥ subunit.

The invention further relates to a methode for the preparation of a pharmaceutical composition, which comprises mixing an oligonucleotide according to the invention with physiologically acceptable exipient and if appropiate suitable additives and/or auxiliaries.

The invention furthermore relates to the use of the oligonucleotides, or of pharmaceuticals which comprise these oligonucleotides, for treating diseases in which the integrin αᵥ subunit or its overexpression is the causative factor or is involved.

The invention relates, to the use of the oligonucleotides or a pharmaceutical composition prepared thereof for the treatment and/or prophylaxis of osteoporosis, for the treatment and/or prophylaxis of cardiovascular diseases, such as restenosis, and for the treatment of cancer, e.g. for inhibiting tumor growth and tumor metastasis. In particular, the invention relates to the use of the oligonucleotides or a pharmaceutical composition thereof for treating cancer or for preventing tumor metastasis or restenosis, or for preparing pharmaceuticals which can be used for treating cancer or for preventing tumor metastasis or restenosis.

The invention furthermore relates to methods of use for treating cancer or for preventing tumor metastasis or restenosis in combination with known therapeutic methods, for example with methods which are currently employed for treating cancer or for preventing tumor metastasis or restenosis. Preference is given to combination with radiation therapy and known chemotherapeutic agents, such as cisplatin, cyclophosphamide, 5-fluorouracil, adriamycin, daunorubicin or tamoxifen.

The invention furthermore relates to pharmaceutical preparations which comprise oligonucleotides and/or their physiologically tolerated salts in addition to pharmaceutically unobjectionable excipients and/or auxiliary substances.

The oligonucleotides and/or their physiologically tolerated salts can be administered to animals, preferably mammals, and in particular humans, as pharmaceuticals on their own, in mixtures with each other, or in the form of pharmaceutical preparations which permit topical, percutaneous, parenteral or enteral use and which comprise, as the active constituent, an effective dose of at least one oligonucleotide in addition to customary pharmaceutically unobjectionable excipients and auxiliary substances. The preparations normally comprise from about 0.1 to 90% by weight of the therapeutically active compound. In order to treat restenosis, preference is given to a topical use, for example in the form of administration using a catheter. In the case of cancer, preference is given to infusions and oral administration, while in the case of osteoporosis, preference is given to oral administration.

The pharmaceutical products are prepared in a manner known per se (e.g. Remingtons Pharmaceutical Sciences, Mack Publ. Co., Easton, PA), with pharmaceutically inert inorganic and/or organic excipients being used. Lactose, corn starch and/or derivatives thereof, talc, stearic acid and/or its salts, etc. can, for example, be used for preparing pills, tablets, coated tablets and hard gelatin capsules. Examples of excipients for soft gelatin capsules and/or suppositories are fats, waxes, semisolid and liquid polyols, natural and/or hardened oils, etc. Examples of suitable excipients for preparing solutions and/or syrups are water, sucrose, invert sugar, glucose, polyols, etc. Suitable excipients for preparing injection solutions are water, alcohols, glycerol, polyols, vegetable oils, etc. Suitable excipients for microcapsules, implants and/or rods are mixed polymers of glycolic acid and lactic acid. In addition, liposome formulations which are known to the skilled person (N.

Weiner, Drug Develop Ind Pharm 15 (1989) 1523; "Liposome Dermatics, Springer Verlag 1992), for example HVJ Liposomes (Hayashi, Gene Therapy 3 (1996) 878) are suitable. Dermal administration can also be effected, for example, using ionophoretic methods and/or by means of electroporation. Furthermore, use can be made of lipofectins and other carrier systems, for example those which are used in gene therapy. Systems which can be used to introduce oligonucleotides in a highly efficient manner into eukaryotic cells or into the nuclei of eukaryotic cells are particularly suitable.

In addition to the active ingredients and excipients, a pharmaceutical preparation can also comprise additives, such as fillers, extenders, disintegrants, binders, lubricants, wetting agents, stabilizing agents, emulsifiers, preservatives, sweeteners, dyes, flavorings or aromatizing agents, thickeners, diluents or buffering substances, and, in addition, solvents and/or solubilizing agents and/or agents for achieving a slow release effect, and also salts for altering the osmotic pressure, coating agents and/or antioxidants. They may also comprise two or more different oligonucleotides and/or their physiologically tolerated salts and, furthermore, in addition to at least one oligonucleotide, one or more different therapeutically active ingredients.
The dose can vary within wide limits and is to be adjusted to the individual circumstances in each individual case.

The invention relates to a pharmaceutical composition which comprises at least one oligonucleotide according to the invention that can be used for the treatment of diseases which are assoziated with abnormal cell proliferation, cell migration, cell differentiation, angiogenesis, retinal neurite outgrowth, bone resorption, phagocytosis, immune response, signal transduction and the metastasis of neoplastic cells. Such pharmaceutical composition can be used for the treatment and prevention of cancer and metastasis of cancer, the treatment and prevention of osteoporosis, the treatment of ocular diseases, chronic inflammation, psorasis, restenosis and the support of wound healing.

More deteiled description of particular aspects of the invention:
Adhesion of cells to substrate has been recognized as a critical event requiring expression of specific receptors capable of recognizing and binding components of the extracellular matrices, the so-called 'cell adhesion molecules' (1). The integrin receptors represent the most relevant cell-surface protein by which cells bind to extracellular matrices. Permutations of the α and β integrin subunits serve as receptors for many adhesive proteins, their interaction with the extracellular matrices being responsible for correct cell growth, survival and function (2) (3).
Osteoclasts are bone resorbing, multinucleated cells usually found in contact with the mineralized bone matrix within lacunae which are the result of their resorptive activity (4). Bone resorption requires the osteoclast to attach to the bone surface and firmly adhere to it to form the sealing membrane, a specialized adhesion area which critically contribute to the maintenance of the controlled composition of the resorption lacuna milieu (5). In this extracellular sealed space, proteolytic enzymes and hydrogen ions degrade both the organic and the inorganic components of bone (6). Due to the importance of the sealing membrane organization to osteoclast function, conditions that interfere with the adhesion properties of the cell modulate the resorptive activity (7).

The vitronectin receptor (integrin aᵥb₃) is present in selected cell types, including osteoclasts where it is highly expressed and plays a functional role related to the resorbing activity (8). The receptor consists of the αᵥ subunit of ~150kDa, and the β₃ subunit of ~95 to 115 kDa (9). Besides vitronectin, aᵥb₃ recognizes the Arg-Gly-Asp (RGD) adhesive sequence motif present in the bone matrix proteins osteopontin and bone sialoprotein. (10,11). As for many α subunits, the αᵥ chain has a major role in determining the ligand specificity (10,12). Besides the β₃ chain it can form heterodimers with at least four other b subunits (β₁, β₅, β₆, and β₈) and has been demonstrated to have several highly conserved regions among species (12). Taken together, this information suggests that the αᵥ integrin subunit may represent an important target for regulation of specific functions associated with the adhesive capacity of cells.

Strategies aiming at the inhibition of αᵥ-mediated cell adhesion may greatly facilitate therapeutical approaches to osteopenic syndromes, as well as providing insights into the mechanisms underlying impairment of osteoclast activity in osteopetroses. Antisense oligodeoxynucleotides (ON s) offer the opportunities for sequence-specific inhibition of gene expression (13-20). Phosphodiester bonded ON s are rapidly degraded in serum and within cells, with half lives of less than two hours (14,15,21,22). However, chemical modification of the naturally occurring phosphodiester bonds, such as replacement of a non-bridging oxygen by sulfur, increases their intracellular stability against nucleases (23,24). It was shown (25) that partial phosphorothioation is equally effective at reducing the amount of target protein and has the additional advantage of decreasing non sequence-related side effects occasionally associated with phosphorothioated molecules (19). In this study, partially phosphorothyoated αᵥ-antisense ON s were used and evidence was obtained that this reagent impairs osteoclast adhesion and bone resorption. Furthermore, this is the first time that αᵥ antisense ON treatment induces apoptosis of rabbit osteoclasts *in vitro,* and that this apoptotic process is accompanied by altered expression of p21^{WAF1/CIP1} and bcl-2 genes.

Integrin-mediated cell anchorage regulates a number of cell functions (1-3), and the αᵥb₃ receptor is known to play a critical role in the control of osteoclast activity (38,39). Surprisingly it was found that specific inhibition of αᵥ integrin expression by an antisense reagent, which reduced both osteoclast adhesion and bone resorption *in vitro,* induced programmed cell death by mechanisms involving the regulation of the p21^{WAF1/CIP1} and the bcl-2, but not the bax genes.

Impairment of adhesion has long been demonstrated to represent an important mechanism for inhibiting bone resorption. In this study it was shown that such inhibition is obtained when the αᵥ integrin subunit synthesis is potently reduced by an antisense mechanism while the b₃ integrin subunit levels remain unchanged. This effect is based on the specificity of Watson-Crick base pairing between the antisense ON and the target mRNA (14,19,22,24,40), which offer the potential to block the expression of specific genes within cells. This has been reported in numerous tissue culture experiments, and in several recent *in vivo* studies (41). Although the mechanism of action of antisense ON s is generally hard to prove (14,16,42,43), in this study reasonable evidence was obtained supporting a specific antisense effect. Firstly, the observed inhibition is sequence-specific in that only the αᵥ-antisense ON is effective, whereas the sense, inverted and mismatch control ON s were not active. Secondly, the observed inhibition is specific for the target protein, since only the αᵥ protein levels were reduced, while b₃ and actin remain unchanged.

The αᵥ antisense ON used in this study spanned the AUG start site, was located between bases 220 and 237 of the human sequence and complemented the rabbit gene. This reagent was found to have a high potency, at least in part determined by a local concentrating effect via adsorption to the substrate, which was greater than that of the AS3 and AS4 αᵥ antisense ON s reported to inhibit melanoma cell adhesion *in vitro* (37).

Antisense DNA approaches have been used to control a number of osteoclast functions (32,44-47). ON s against the 16 kD and 60 kD subunits of the V-ATPase have been demonstrated to inhibit at high doses (approximately 30 mM) rat osteoclast bone resorption and polarization (44,45), shown microscopically by disruption of the F-actin ring structure that is typically observed in resorbing osteoclasts (2,48). The 5543 αᵥ antisense ON showed no obvious effects on the F-actin ring structures (not shown), but clearly induced programmed cell death, an event which has been demonstrated to rapidly disrupt the cells (49,50).

Adhesion to the substrate has long been considered critical to survival of cells which grow adherent to an extracellular matrix (3). Anchorage-dependent growth has been demonstrated in a variety of cell types to modulate function and gene expression (51, 52). The transition from anchorage-dependent to anchorage-independent growth is a characteristic feature of neoplastic transformation, normal cells being dependent upon interaction with the substrate for physiological behavior. Apoptosis is a highly conserved active cellular mechanism characterized by cell shrinkage, chromatin condensation and nuclear fragmentation (49). All these events have been observed in our cells treated with the αᵥ antisense ON , thus suggesting that alteration of the interaction with the substrate may control the size of the osteoclast population *in vitro.*

Programmed cell death is regulated by a number of genes which control the balance of cell proliferation and cell death (49,50). A cell undergoes apoptosis as a result of information received by the microenvironment and interpreted by the intracellular machinery. Cell-surface receptor-mediated mechanisms which control apoptosis often act through signal transduction systems involving the activation of second messengers regulating the transcription of specific genes (49,50). For example, the p21^{WAF1/CIP1} gene encodes for a protein which inhibits the cyclin/cyclin-dependent kinase, CDK (53). Ligation of aᵥb₃ during angiogenesis has been demonstrated to suppress the expression of p21^{wAF1/CIP1} and to stimulate cell survival (54). In our study we found that inhibition of aᵥ integrin synthesis by the 5543 antisense ON stimulated the expression of p21^{WAF1/ClP1} in a mixed population enriched in osteoclasts and osteoclast precursors. This gene was clearly detectable in untreated osteoclasts, as expected for post-mitotic cells, as well as in a small number (9%) of mononuclear cells. However, its immunodetection in osteoclasts was enhanced and, at the same time, the number of mononuclear cells expressing the gene was found to be increased in αᵥ antisense- relative to control ON -treated cultures. Inhibition of cyclin-CDK complex by p21^{WAF1/CIP1} interferes with phosphorylation events critical to cell cycle transition (53). It has recently been suggested that this cell cycle inhibitor is implicated in growth arrest associated with terminal differentiation (55). Furthermore, potential roles of this gene in events leading to efficient repair of DNA damage or to apoptosis (56,57) has been hypothesized. Expression of the p21^{WAF/ClP1} protein has been found to be suppressed in endothelial cells during αᵥb₃-mediated adhesion both *in vivo* and *in vitro* (52), and stimulated in response to disruption of fibroblast-to-extracellular matrix interaction (58). These data indicate a potential direct link between integrin-dependent anchorage and regulation of p21^{WAF1/CIP1} to promote cell survival.
Among the numerous genes that have been implicated in the control of cell survival, the bcl-2 gene, which encodes two splice variants, Bcl-2a and Bcl-2b, is known to be a negative regulator of cell death (49,50). The bcl-2 prolonges the survival of non-cycling cells, and prevents the death by apoptosis of cycling cells (49,50). In contrast, the bax gene encodes a protein that has a role in opposition to Bcl-2, acting as a death promoting factor. Bcl-2 is known to potentiate cell survival based on its ability to dimerize with Bax (49,50); therefore a high bcl-2/bax ratio promotes cell survival, whereas a low bcl-2/bax ratio induces apoptosis. This study clearly demonstrates that osteoclast programmed death induced by impairment of αᵥ₋dependent anchorage, is associated with a dramatic reduction of the bcl-2 expression rather than with changes in bax levels. These events lead to a clear lowering of the bcl-2/bax ratio which is likely to induce apoptosis in osteoclasts and their putative precursors. In this context it is interesting to note that Strömblad et al. (54) recently demonstrated that denied attachment of endothelial cells on a bovine serum albumin-coated surface affected expression of both genes, inducing a decrease of bcl-2 and an increase of bax relative to cells attached to immobilized anti-αᵥb₃ antibody. These findings indicate that inhibition of αᵥ integrin dependent anchorage in different cellular models may induce distinct effects on the expression of the bcl-2 and the bax gene products which, lead to a reduction of the bcl-2/bax ratio.

In conclusion, this study provides evidence that an antisense ON targeted to the αᵥ integrin subunit mRNA of osteoclasts successfully inhibits adhesion and bone resorption with high specificity and potency, and that the αᵥ-dependent anchorage is mandatory for the survival of osteoclasts and their putative precursors. A clear link between inhibition of αᵥ-mediated adhesion and regulation of p21^{WAF/CIP1} and bcl-2/bax ratio has been demonstrated to represent the underlying pathway that promotes apoptosis in the rabbit osteoclast lineage. The development of this approach opens an avenue to alternative therapies for bone diseases.

The most common malignant tumours frequently involve the skeleton, e.g. advanced breast carcinomas (59). In the United Kingdom breast cancer affects between 7 and 10 % of women and globally accounts for one fifth of female malignancies, and at least half of these patients will die from metastatic disease (60). Bone metastasis is apparently associated with most primary tumours, although, there are some malignancies which are predisposed to spread to the skeleton. In particular, prostrate cancer and breast carcinoma almost always metastases to bone (59, 60). Therefore, elucidation of the cellular mechanisms involved may aid in the development of new, effective treatments which could prevent an essentially incurable and catastrophic disease.

The spread of cancer requires the disengaging of cells from a primary tumour site and migration to and attachment at a secondary settlement location. Many workers have shown that a number of cell adhesion molecules play a role in metastasis and that integrins are especially involved in tumourigenic spread (61).

Integrins are not only implicated in cell-cell and cell-ECM (ECM = extracellular-matrix) interactions but also signaling into the cell, and are thus involved in sensing the cellular microenvironment, playing key roles in cellular activities such as migration, differentiation, survival and tissue (re)modeling in both normal and pathological states (64, 65).

In the case of breast cancer cell lines there is considerable evidence of altered integrin levels in the tumourigenic situation in comparison to the native, parental background. αᵥ integrins, and especially the vitronectin (αᵥβ₃), are highly expressed and could have a prominent role in breast carcinoma metastasis to bone (66). A number of breast carcinoma cell lines are available for studying the effect of various anti-adhesive treatments, such as monoclonal antibodies and peptides, and these include the MDA-MB cell line series. In a study by Meyer and colleagues (66) where they assessed the expression of αᵥ integrins in eight different breast cancer cell lines, they found that the classical αᵥβ₃, vitronectin receptor was only expressed in MDA-MB231 cells whereas αᵥβ₅ is expressed by all breast cancer cells and αᵥβ₁ is expressed on the majority. The αᵥ subunit could be a key target for anti-cancer therapies. Since the vitronectin receptor has been shown to be crucial in tumour progression and expression of the invasive phenotype (66, 67) as shown in the melanoma cell types (68, 69). Therefore, there is considerable interest in trying to modify breast cancer integrin expression profiles with consequential effects upon primary tumour growth and spread.

The therapeutic potential of an antisence ONs when designed to suppress the cellular function of the αᵥ integrin subunit in breast cancer cells was shown. Extensive analysis of MDA-MB231 (human breast carcinoma cell line) cell line physiology challenged with low doses of antisense ONs specific for integrin αᵥ subunit showed efficient inhibition of cell-to ECM (extracellular Matrix) adhesion. These anti-adhesive effects are due to decreases in the level of total αᵥ protein and result in activation of programmed cell death.

ON 5543 induced an increased apoptosis rate. It is known that inhibition of anchorage to substrate activates intracellular signals to apoptosis, and that the αᵥ receptors play a pivotal role in preventing this event (74-77). Apoptosis is an active process requiring transcriptional events prior to nuclear fragmentation and packaging of DNA into "apoptotic bodies" (78). There are several cellular "switches" activating cellular death, one of which is the p53 protein, a gene product that senses the DNA damage and arrests G₀→G₁ cell cycle transition through activation of the p21^{WAF1/CIP1} inhibitor of the cyclin-cyclin dependent kinase complex (75, 79). Other signals to apoptosis are represented by reciprocal changes of the Bcl-2 and Bax gene products. When the Bcl-2/Bax ration is reduced, the pro-apoptotic performance of the Bax product is favoured. In the study with MDA-MB231 cells the expression of these genes was unaltered by ON5543. However, the p53 protein was translocated from the cytoplasm to the nucleus, an event which occurs as a consequence of interruption of αᵥ-mediated adhesion in other cellular models (75). The p53 expressed by the MDA-MB231 cell line is a mutant form (79), and its signalling pathway is not fully understood.

The αᵥ-integrin receptors proved to be relevant for both osteoclast physiology and breast cancer dissemination to bone (68, 69). Other means have already been used for disrupting osteoclast adhesion to substrate. The include αᵥβ₃ neutralizing antibodies, the snake venom disintegrin echistatin and RGD peptides (71, 72). Other workers have recently shown the advantage of using a non-peptide small molecule mimetic (SC-68448) to antagonise the αᵥβ₃ integrin (80). Compared to RGD peptides ON 5543 is highly specific for αᵥ and, at variance with the antibodies and non-peptide mimetics, it blocks all the αᵥ receptors, regardless of the associated β subunit. Besides the αᵥβ₃ receptor, osteoclasts and breast carcinoma cells also express the αᵥβ₁ and the αᵥβ₃ (66, 71, 72) receptors. Therefore, ON 5543 targeted to the αᵥ gene (mRNA respectively) offers the advantage of blocking a mechanism shared by both cell types involved in the metastatic lesions, with high efficiency and specificity for all the αᵥ receptors.

Despite the noticeable similarity of effect on adhesion, the mechanism underlying the apoptotic events activated by ON 5543 in osteoclasts and MDA-MB231 cells seem to differ in some specific aspects. Osteoclasts showed an increase of p21^{WAF1/CIP1} expression and a reduction of the Bcl-2/Bax ratio (77), whereas none of these genes were modified in MDA-MB231 cells. This indicates that the intracellular signals to apoptosis regulated by the αᵥ gene is heterogeneous maybe cell type specific.

ON 5543 also induces apoptosis with a mechanism probably involving the p53 (but no changes in p53 expression were observed, but a p53 translocation from the cytasol to the nucleus was observed), but independent of the p₂₁^{WAF1/CIP1} cyclincyclin dependent kinase complex inhibitor, of the cell survival factor Bcl-2 and the pro-apoptotic factor Bax. The efficacy of the ON 5543 in interrupting the contact with ECM of tumour cells and osteoclasts suggests that this compound may block metastatic bone diseases.

### Examples:

### Abbreviations:

| | |
|---|---|
| BSA: | Bovine Serum Albumin. |
| DMEM: | Dulbecco's modified Minimum Essential Medium. |
| ECL: | Enhanced ChemiLuminescence. |
| FBS: | Fetal Bovine Serum. |
| ON : | oligodeoxynucleotides. |
| PBS: | Phosphate Buffer Saline. |
| TRAP: | Tartrate-Resistant Acid Phosphatase. |
| TUNEL: | TdT-mediated dUTP-biotin nick end labeling. |

### Methods

### Reagents.

Cell culture media, reagents and sera were from Hyclone (Röntegenstraat, Holland) or Gibco Lifesciences (Inchinnan, UK). Culture dishes and sterile glass ware were from Falcon Becton-Dickinson (Lincoln Park, NJ) and from Costar Co. (Cambridge, MA). Apoptosis/DNA fragmentation detection kit (TUNEL) was from Chemicon (Tamecula, CA). Chemicais, of the purest grade, were from Sigma Chemical Co. (St. Louis, MO). The human αᵥβ₃, 23C6 monoclonal antibody (26) and αᵥ, 13C2 monoclonal antibody was produced in our laboratory. The human αᵥ monoclonal antibody (clone #139) and the human β₃ polyclonal antibody (clone #1264) (27), were obtained from the Department of Dermatology, State University of New York at Stony Brook, New York. The anti-p21^{WAF1/CIP1} (Ab-5) and p53 (OPO9) antibodies were from Oncogene Research Calbiochem (Cambridge, MA). The anti-Bcl-2 (sc 492), the anti-Bax (sc 493), the anti-actin (sc 1616) and the secondary antibodies were from Santa Cruz Biotechnology Inc. (Heidelberg, Germany). ECL kit was from Amersham International plc (Little Chalfont, U.K.). 1,25-dihydroxy vitamin D₃ was obtained from Roche S.P.A. (Milan, Italy).

### RT-PCR:

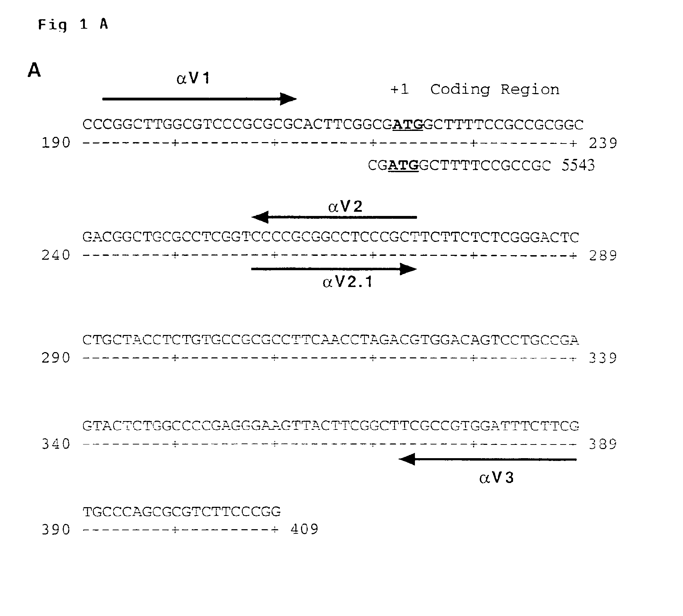

Total RNA was prepared from rabbit spleen and bone marrow, high αᵥ expressing tissues. The two flanking primers, αᵥ₁ and αᵥ₃, amplified a predicted band of 198 bp (see Fig. 1). The internal α_{v2.1} and the 3' αᵥ₃ primers amplify a band of 133 bp, though this product would not include the ATG start site. Therefore, nested PCR using the αᵥ₁ + αᵥ₃ product as template, utilizing the primers αᵥ₁ and αᵥ₂, were used to amplify an 82 bp band which should include the start site sequence. This was T:A subcloned into pCR2.1 and sequenced automatically in the forward and reverse directions.

### Cells:

Freshly isolated osteoclasts were obtained from newborn (4-7 days) New Zealand White rabbits as described by Chambers et al. (30) and Caselli et al. (31). Cells were allowed to attach to substrates for 45-90 min, then incubated in DMEM supplemented with 10% FBS, 100 lU/ml penicillin, 100 µg/ml streptomycin, in a water-saturated atmosphere of 95% air and 5% CO₂.
Rabbit osteoclasts were also differentiated *in vitro* from bone marrow. Bone marrow was flushed from long bones and cultured as described above. After 24 hours, non-adherent cells were removed by aspiration and repeated washes, and the total adherent cell fraction was incubated with 10 nM 1,25-dihydroxy vitamin D₃ for ten days. At the end of incubation, contaminating stromal cells were removed by mild trypsinization and osteoclast phenotype was evaluated by positive staining for TRAP activity, retraction in response to 100 n M salmon calcitonin, and resorption pit formation.
Mouse osteoclasts were generated *in vitro* and characterized as described by Tanaka et al. (32).

The MDA-MB231 human breast carcinoma cell line was acquired from the ICRF mammalian cell culture laboratory and the ETCC. Cells were cultured in Dulbecco's Modified Minimum Essential Medium (DMEM) with Earle's salts containing 10 % foetal calf serum (FCS), 100 U/ml penicillin, 0.2 mg/ml streptomycin, 0.2 % glycine at 37°C in 5 % CO₂, 95 % air in an humidified atmosphere.

### Treatment of MDA-MB231 cells with ON's

ONs were diluted in DMEM in stock solutions and stored in aliquots at -20°C until use. Treatment of MDA-MB231 cells was performed in the presence of the uptake enhancer lipofectamine (5 µg/ml). Lipofectamine was mixed with appropriate dilutions of ONs and preincubated for 30 minutes prior to administration to the cells.

### Substrates:

Osteoclasts were plated either onto glass, bone or dentine. 10% FBS-containing DMEM was used as a standard adhesion substrate during cell culture on artificial substrates. Alternatively, glass coverslips were previously coated with adhesive proteins and cells were then incubated in low serum (1 %)-containing DMEM. For substrate coating, 20% FBS or 10µg/ml fibrinogen, fibronectin, vitronectin and fibrillar collagen were used as sources of adhesive substrates. Wells were coated with the proteins and incubated 3 hours or overnight at 4°C. The solutions were then replaced with 60% methanol, and the wells were further incubated for 1 h at 4°C. Methanol was removed by aspiration, and the wells were incubated with 100 µl of a buffer containing 50 mM Tris-HCl (pH 7.8), 110 mM NaCl, 5 mM CaCl₂, 1% BSA, and 0.1 mM phenylmethylsulfonylfluoride for 30 min at room temperature. Finally, wells were washed three times with DMEM supplemented with 0.2% BSA and immediately used for the experiment.

### Treatment with ON s:

ON s were dissolved in water and diluted in DMEM as stock solutions and stored in aliquots at -20°C until used. Treatment of osteoclasts was performed by incubating the cells with appropriate concentrations of the ON s for different periods.

### FITC-ON labeled analysis:

FITC-labeled ON were synthesized in order to monitor their uptake into the cells and adsorbance to substrates. Cells, were incubated for 24 hours with 1 µM FITC-ON s, fixed and observed by confocal fluorescence microscopy (Leica Tcs-NT system). For adsorption to glass or dentine, ranging concentrations of FITC-ON (0.2-20 µM) were incubated together for up to 24 hours.

### Apoptosis:

Cell morphology was evaluated by phase contrast microscopy. Bisbenzimide, which specifically binds to the Adenine-Thymidine regions of DNA, was used for nuclear staining. Cells were fixed in Carnoy's fixative (methanol-glacial acetic acid 3:1), incubated for 30 min in 0.5 µg/ml bisbenzimide, rinsed (2x in distilled water), mounted in glycerol-PBS 1:1 and observed by conventional epifluorescence microscopy.
To evaluate fragmented DNA the TUNEL method (34) was used according to manufacturer's instruction. Briefly, cells were fixed in 4% buffered paraformaldehyde, washed (2 min, 4x in distilled water) and incubated in TdT buffer for 5-10 min at room temperature. Buffer was then removed, and cells were incubated in TdT- and Biotin-dUPT-containing buffer for 60 min at 37°C. Cells were then washed in TdT buffer for 15 min at room temperature, washed (2 min, 4x in distilled water), blocked in blocking reagent and incubated with avidin-conjugated FITC for 30 min at 37°C.
Cells were washed in PBS (5 min, 3x), counterstained with 0.5 µg/ml propidium iodide, washed in PBS (3 min), mounted and observed by conventional epifluorescence microscopy.

### Statistics:

Data are the mean+SE from at least three independent experiments. Statistical analysis was performed by one-way analysis of variance (ANOVA) followed by the Student's *t* test and by the Mann-Whitney test. A p value < 0.05 was considered statistically significant.

### Example 1: Oligodeoxynucleotide synthesis.

The αᵥ ON sequences and the rabbit AUG start site sequence.
Several of the sequences of the ON s used in this study are shown in Table 3. Partially phosphorothioated antisense ON s directed to the αᵥ subunit of human vitronectin receptor were chosen on the basis of the BLAST alignment program (35). Alignment of the known species variants for the αᵥ subunit revealed high homologies around the translational start site (Fig. 1A).
Since rabbit osteoclasts were used in the following studies, the rabbit translational start sequence was determined by RT-PCR. Three pairs of degenerate primers were designed, that when used in combination, reveal the rabbit sequence (Fig. 1B and C). Fig. 1D represents the rabbit αᵥ translational start sequence, including the ON 5543 antisense sequence. The sequences are highly homologous (98.9%) with only one base discrepancy at position 83 where the rabbit αᵥ contained a thymidine base compared with a cytosine in the human sequence (36).

ON s were synthesized using an Applied Biosystems 394 DNA synthesizer (Perkin Elmer Applied Biosystems, Inc., Foster City, USA) and standard phosphoramidite chemistry. After coupling, phosphorothioate linkages were introduced by sulfurization using the Beaucage reagent (28) followed by capping with acetic anhydride and N-methylimidazole. After cleavage from the solid support and final deprotection by treatment with concentrated ammonia, ON s were purified by polyacrylamide gel electrophoresis. The C-5 propynyl pyrimidine modified ON s were prepared as described previously (29). All ON s were analyzed by negative ion electrospray mass spectroscopy (Fisons Bio-Q) which in all cases confirmed the calculated mass. The C16-modified oligonucleotides were synthesized using hexadecyloxy (cyanoethoxy) N,N-diisopropyl aminophosphane as phosphitylating reagent in the last step of oligonucleotide synthesis in place of a standard amidite.
Analysis of the oligonucleotides was done by
a) Analytical gel electrophoresis in 20% acrylamide, 8M urea, 45µM tris-borate buffer, pH 7.0 and/or
b) HPLC-analysis: Waters GenPak FAXcolumn, gradient CH₃CN (400ml), H₂O (1.61), NaH₂PO₄ (3.1g), NaCl (11.7g), pH6.8 (0.1 M an NaCl) after CH₃CN (400ml), H₂O (1.6l), NaH₂PO₄ (3.1 g), NaCl (175.3g), pH6.8 (1.5M an NaCl) and/or
c) capillary electrophoresis using a Beckmann capillary eCAP^{TM}, U100P Gel Column, 65 cm length, 100 mm I.D., window 15 cm from one end, buffer 140 µM Tris, 360mM borate, 7M urea and/or
d) negativ ion electrospray mass spectrometry which in all cases confirmed the expected mass values.

Following oligonucleotides against (human) HSVTNR mRNA were prepared:
ON 5541 (antisense)
   3'-G*A*AGC*C*GC*TAC*C*GAAAAG*G*C- 5'
ON 5542 (antisense)
   3'-C*G*C*GT*GAAG*C*CGC*TA*C*C*G- 5'
ON 5543 antisense
   3'-G*C*T*AC*CGAAAAGG*CGG*C*G - 5'
ON 5043 (inverted contr. of ON 5543)
   5'-G*C*T*AC*CGAAAAGG*CGG*C*G-3'
ON 5044 (sense of ON 5543)
   5'-C*G*ATGGC*TT*TT*CCGCC*G*C-3'
ON 5045 (mismatch of ON 5543)
   3'-G*C*T*GC*CGAGAGAG*CAA*C*G-5'
ON 5959 antisense
   5'- C*G T*C*T A G G T*T*G A A G G*C*G -3'
ON 5972 inverted Control
   5'- G*C*G G A A G*T*T G G A T*C*T G*C -3'
ON 5970 sense
   5'-C*G C*C T*T*C A A C*C*T A G A*C*G -3'
ON 5971 mismatch
   5'- C*G T*C*T G A G T*T*G A G A G*C*G -3'
ON 5431 antisense
   3'- G*C*T*A C* C G A A A A G G*C G G*C*G - C16 -5'
ON 5432 inverted repeat
   5'- G*C*T*AC*CGAAAAGG*CGG*C*G-C16 -3'
ON 5504 antisense
   3'- G*C*T*A C* C G A A A A G G*C G G*C*G -5'
ON 5503 inverted repeat
   5'- G*C*T*A C* C G A A A A G G*C G G*C*G -3'
ON 5506 antisense
   3'- G*C*T*A C* C G A A A A G G*C G G*C*G - C 16 -5'
ON 5505 inverted repeat
   5'-G*C*T*A C* C G A A A A G G*C G G*C*G -C16- 3'
ON 5959 antisense
   3' G*C*G G A A G*T*T G G A T*C*T G*C 5'

For comparative animal studies a mouse specific antisense oligonucleotide was synthesized:
ON 5468 mouse α_{V} antisense
   3' G*C*T A C*C G A C*G A G G G C*C*C*G 5'
* is a phosphorothioate residue, and C is Propinyl-dC, T is Propinyl-dU

### Example 2: Uptake of the ON 5543 antisense ON and αᵥ protein reduction.

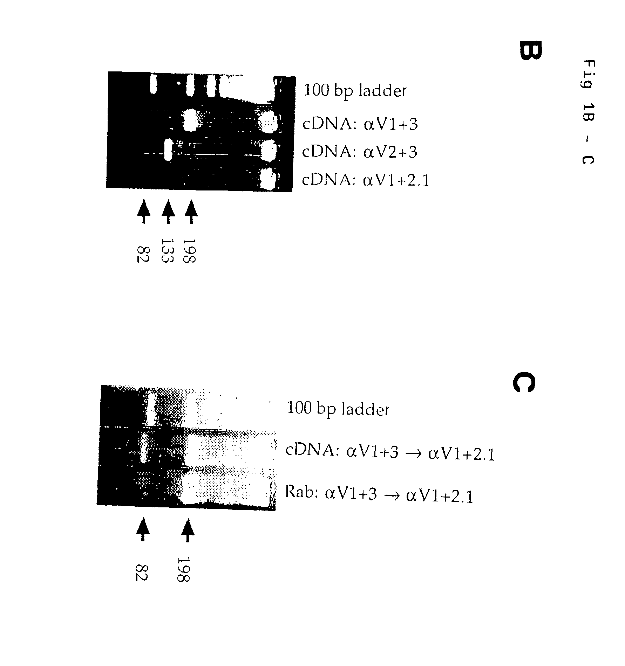

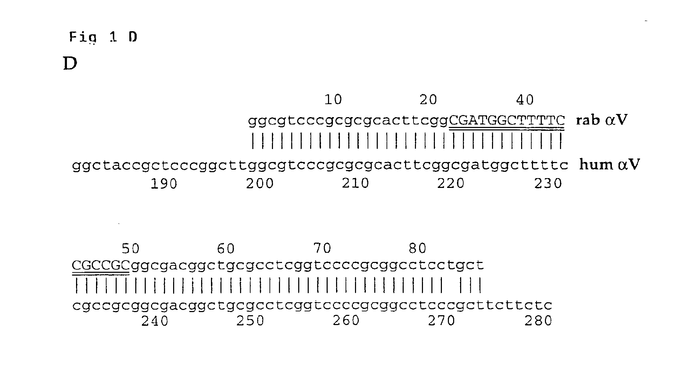

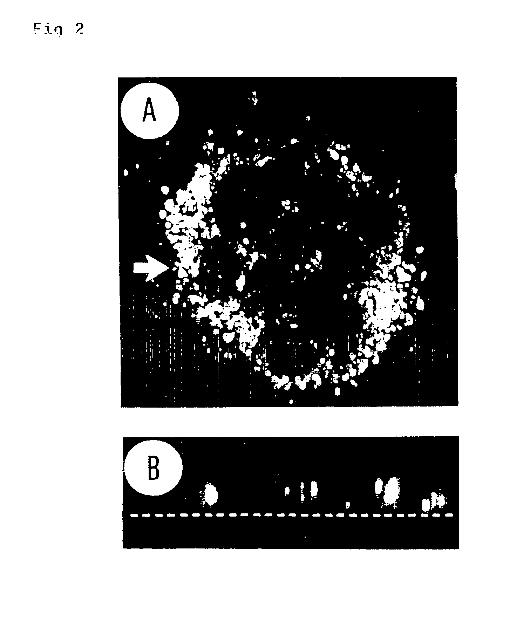

Uptake studies were first performed to examine whether the ON 5543 αᵥ antisense linked to FITC was taken up into osteoclasts. *Ex vivo* rabbit osteoclasts, plated on glass (Fig.2) or dentine (not shown), internalized the fluorescent ON, with the highest levels observed in numerous intracellular vacuoles (Fig. 2); nuclei generally showed low uptake.

To examine whether the antisense ON reduced αᵥ integrin synthesis, large numbers of rabbit osteoclasts were generated *in vitro* from the adherent bone marrow cell fraction cultured for 10 days in the presence of 10 nM 1,25-dihydroxy vitamin D₃. TRAP-positive multinucleated osteoclasts fully active in resorbing bone and retracting in response to 10⁻⁷ M salmon calcitonin appeared by the 6th day of culture and progressively increased with time. Cultures were then cleaned from contaminating stromal cells by mild trypsinization, and those populations containing >80% of cells of the osteoclast lineage (both mature osteoclasts and TRAP-positive putative mononuclear precursors) were treated with the ON 5543 for 48 hours.

### Example 3: Immunoblotting.

Cell extracts from 90% confluent cultures were prepared with TBS buffer (10 mM Tris-HCl, pH 7.4, and 0.9% NaCl) containing 0.5% Triton X-100 and protease inhibitors. Lysates were maintained at 4°C throughout the procedure. Lysates were centrifuged for 10 min at 11,000 g to remove nuclei and cell debris, TCA-precipitated and then solubilized in Laemmli buffer. 30 µg cell protein was subjected to SDS-PAGE (8% acrylamide gel for αᵥ, β₃ and actin and 12% for p21 ^{WAF/CIP1}, bcl-2 and bax), and proteins were transferred onto nitrocellulose filters.

Filters were then incubated with the primary antibody at 4°C overnight, washed and incubated with horseradish peroxidase conjugated secondary antibody at room temperature for 90 min, washed and detected by ECL.

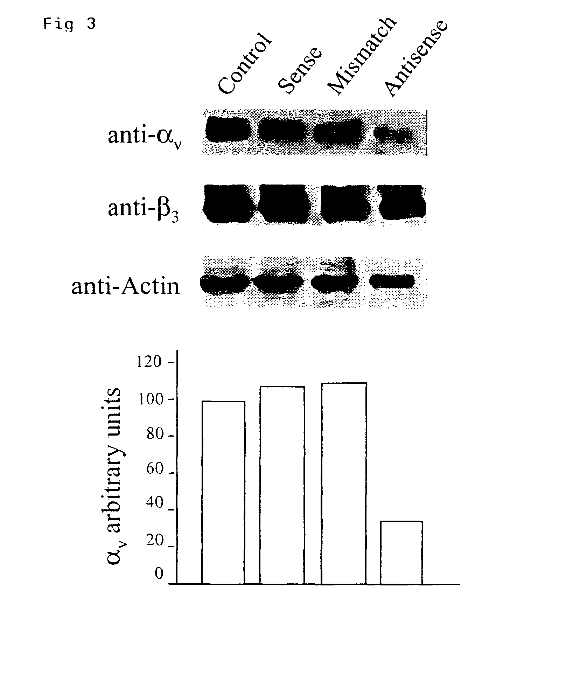

Immunoblotting analysis (Fig. 3A) demonstrated that 1 µM ON 5543 antisense reduced the level of αᵥ protein compared to untreated cultures and to cultures treated with the control ONs. In contrast, no significant change in the β₃ integrin subunit or actin expression was observed. Similar results were obtained using mouse osteoclasts generated *in vitro* as described by Tanaka et al. (32) and treated with mouse-specific αᵥ antisense and control ONs (data not shown).

### Example 4: Cell Adhesion Measurement.

Osteoclasts were plated on 13 mm diameter round coverslips or 4x4 mm bovine bone slices or dentine discs (6 mm diameter) from elephant ivory, and incubated in the presence of ON s for different times. At the end of incubation, non-adhering cells were removed by extensive washing in PBS (3x), and attached cells were fixed in 3% paraformaldehyde in PBS for 15 min at room temperature, rinsed in PBS (3x), stained for TRAP enzyme and observed by phase contrast microscopy. Adhesion was evaluated counting the number of TRAP-positive multinucleated osteoclasts per each coverslip or bone /dentine slice.

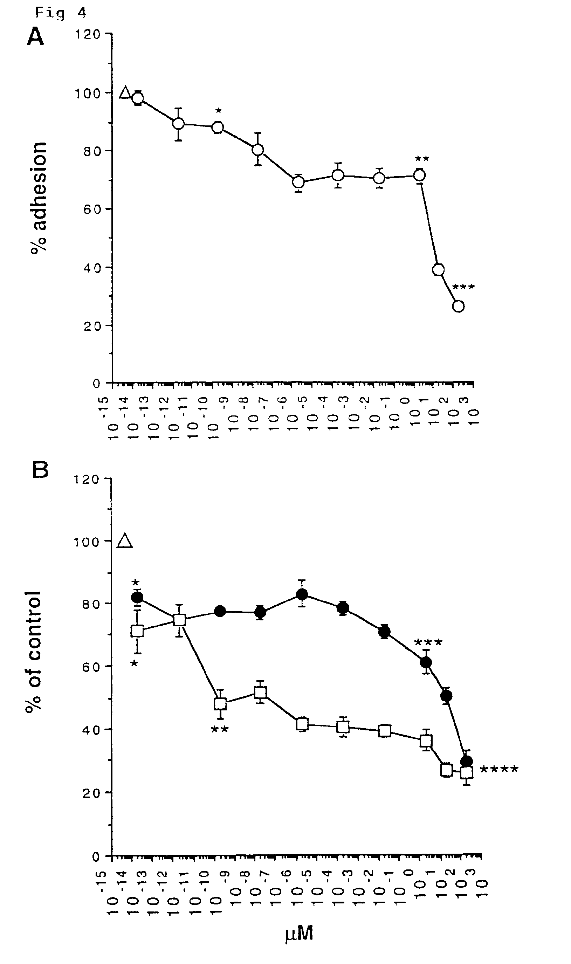

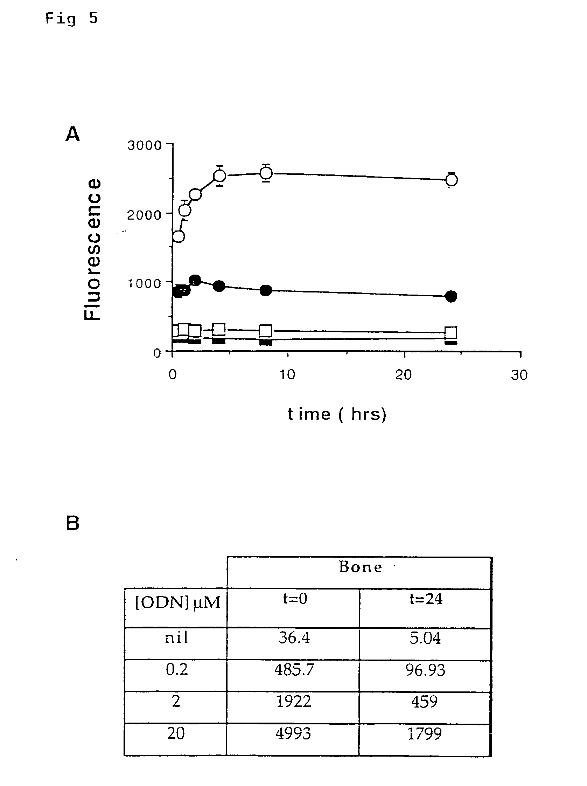

The ON 5543 αᵥ antisense inhibited ex *vivo* rabbit osteoclast adhesion to glass and to bone or dentine, and bone resorption. The titration curves were biphasic and suggested that activity can be seen at concentrations as low as 2x10⁻¹⁰ µM (p<0.05, Fig. 4). This high potency was likely to be due to a local concentrating effect on the substrate support. In fact, a FITC modified ON 5543 (FITC labeled ON 5543) was rapidly adsorbed to the dentine matrix, rising to a maximum at 4 hours (Fig. 5). Similar results were obtained for the FITC-ON 5543 mismatch ON adsorption to dentine and glass (not shown). Likewise, adsorption to glass increased with time and ON concentration (not shown). However, the relative amounts of FITC-labeled ON adsorbed were approximately 30 fold less than that observed with dentine.

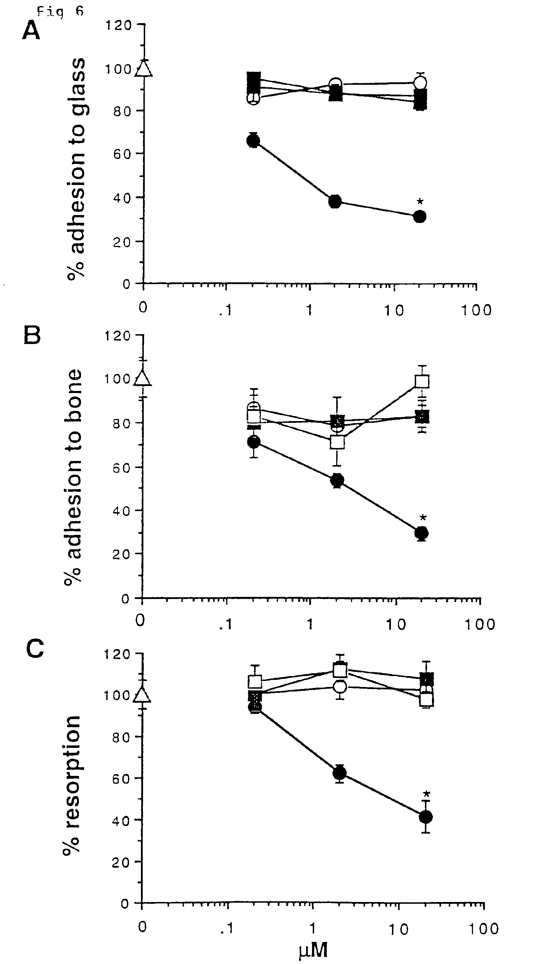

Fig. 6 shows that all three control ONs were ineffective (p=ns) at inhibiting *ex vivo* osteoclast adhesion and resorption relative to the 5543 antisense ON (p<0.0001). Comparative study showed a greater activity of the 5543 antisense ON (20 mM, adhesion 68% and resorption 65% inhibition vs. nil) relative to the AS3 (20 mM, adhesion 53% and resorption 50% inhibition vs. nil) and AS4 (20 mM, adhesion 48% and resorption 50% inhibition vs. nil, n=3-8; p<0.001) αᵥ antisense ONs that were shown to inhibit melanoma cell adhesion in culture (37).

### Example 5: Bone resorption measurement.

Bone resorption was evaluated according to Prallet et al. (33). Osteoclasts were plated onto bone or dentine slices and incubated in the continuous presence of the ONs for 24-48 hours. At the end of incubation, adherent TRAP-positive multinucleated cells were counted under light microscopy, then removed by sonication for 2 min in 0.01 % NaOCl. Slices were rinsed twice in distilled water (20 min), and stained for 4 min in 1 % toluidine blue in 1 % sodium borate, and observed by conventional light microscopy with a 20x objective. The number of resorption pits in at least 5 bone slices or dentine discs for each point was counted in three visual categories, according to Prallet et al., (33). For each experiment, the mean control (vehicle only) value of the pit area index was used as a base to calculate each value as a percentage of the mean control value (31). Alternatively, bone resorption was enumerated as pit number per osteoclast, and normalized to control values of resorption in the presence of vehicle only.

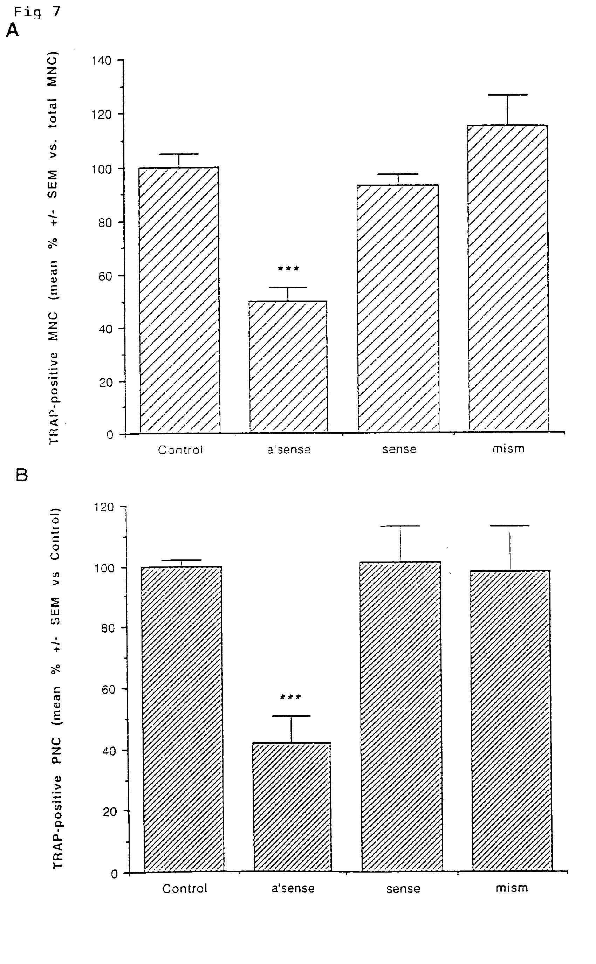

Treatment of osteoclasts generated *in vitro* with the ON 5543 antisense, but not control, ONs showed inhibition of adhesion and resorption equivalent to that observed with ex *vivo* osteoclasts (data not shown). When this fraction was cultured during the 10 days necessary for differentiation in the continuous presence of ON 5543 antisense ON , a significant reduction not only of TRAP-positive multinucleated cells, but also of TRAP-positive putative osteoclast mononuclear precursors was observed. Again, all control ONs were ineffective (Fig. 7). These results indicate efficacy of the antisense ON 5443 also at the early stage of osteoclast development and validate the molecular analysis performed in this study using this cell population.

### Example 6: Substrat specificity

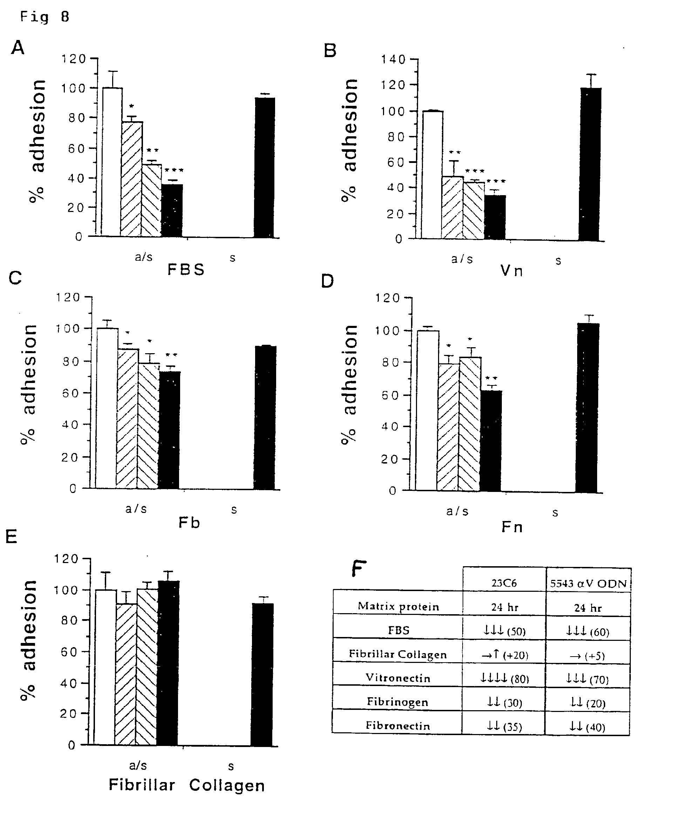

The substrate-specificity of the ON 5543 (abbreviation 5543) effect on osteoclast adhesion was determined (Fig. 8). A concentration-dependent effect on adhesion to serum and vitronectin (Fig. 8A and B) was observed (with 63% inhibition at 20 µM, p<0.001). In the case of adhesion to fibrinogen and fibronectin (Fig. 8C and D) there were small, but statistically significant (p<0.01), reductions in the number of osteoclasts attached after 24 hours, whereas adhesion to fibrillar collagen was unaffected (Fig. 8E, p=ns). The effect of ON 5543 antisense on osteoclast adhesion was compared with that of the function blocking monoclonal antibody 23C6 (to human αᵥβ₃, but cross reactive to the rabbit receptor) (Fig. 8F). The inhibitory effect of 23C6 was most prominent on cells plated onto serum and vitronectin, with the responses to ON 5543 paralleling that of 23C6 on all matrix proteins tested.

### Example 7: Determination of Apoptosis.

Cell morphology was evaluated by phase contrast microscopy. Bisbenzimide, which specifically binds to the Adenine-Thymidine regions of DNA, was used for nuclear staining. Cells were fixed in Carnoy's fixative (methanol-glacial acetic acid 3:1), incubated for 30 min in 0.5 µg/ml bisbenzimide, rinsed (2x in distilled water), mounted in glycerol-PBS 1:1 and observed by conventional epifluorescence microscopy.

To evaluate fragmented DNA the TUNEL method was used according to manufacturer's instruction. Briefly, cells were fixed in 4% buffered paraformaldehyde, washed (2 min, 4x in distilled water) and incubated in TdT buffer for 5-10 min at room temperature. Buffer was then removed, and cells were incubated in TdT- and Biotin-dUPT-containing buffer for 60 min at 37°C. Cells were then washed in TdT buffer for 15 min at room temperature, washed (2 min, 4x in distilled water), blocked in blocking reagent and incubated with avidin-conjugated FITC for 30 min at 37°C. Cells were washed in PBS (5 min, 3x), counterstained with 0.5 µg/ml propidium iodide, washed in PBS (3 min), mounted and observed by conventional epifluorescence microscopy.

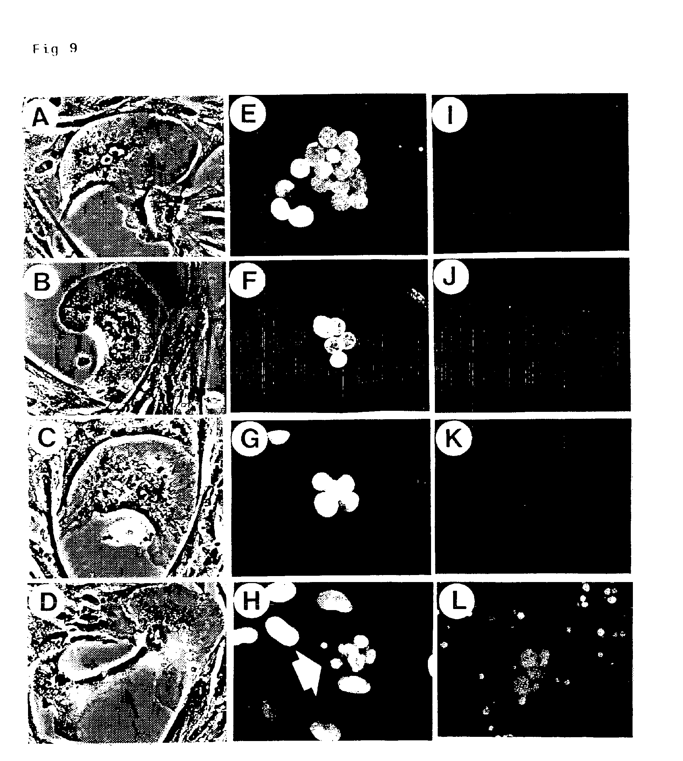

Morphological analysis of ON 5543 antisense ON -treated osteoclasts demonstrated cell retraction relative to control ON s (Fig 9A-D) with nuclei often showing morphological changes consistent with nuclear damage. Therefore, DNA was decorated with bisbenzimide, a compound which specifically binds the adenine-thymidine regions. After 24 h challenge by the antisense ON , nuclei appeared altered with apoptotic bodies were evident (Fig. 9H). Control treated cultures (Fig. 9F and G) showed no alteration, since nuclei were undistiguishable from those in the untreated control (Fig. 9E). To confirm apoptosis, TUNEL staining of fragmented DNA, which reveals programmed cell death at a very early stage, was performed. It was found that in ON 5543 ON -treated cells (Fig. 91) the number of apoptotic nuclei was increased relative to untreated cells or to cells treated with control ON s (Fig. 9J).

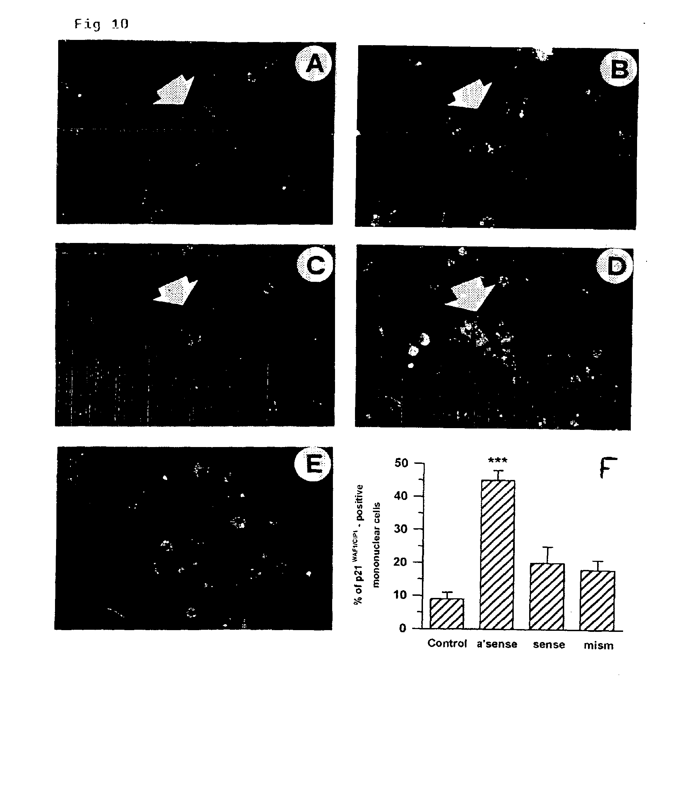

In order to examine the intracellular mechanism inducing apoptosis, experiments were then designed to investigate the genes that were altered to promote cell death upon inhibition of aᵥ synthesis. Immunofluorescence detection of the p₂₁^{WAF1/CIP1} protein demonstrated an increase of expression in both the mononuclear cell fraction and the osteoclasts treated with the ON 5543 αᵥ antisense, but not with control ON s (Fig. 10).

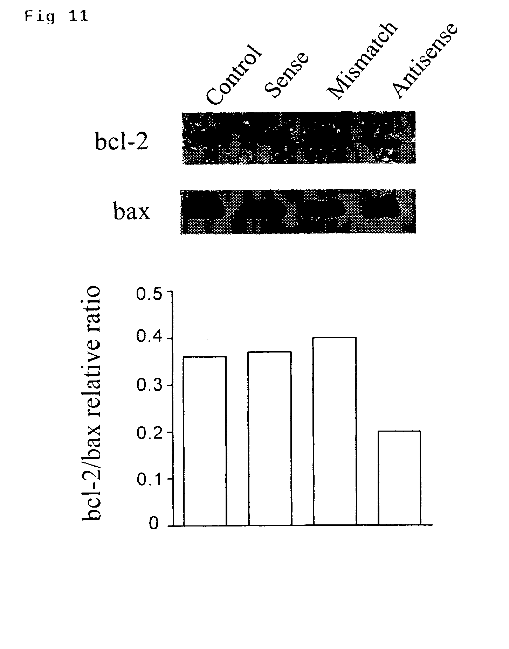

Furthermore, immunoblotting analysis demonstrated that the ON 5543 aᵥ antisense induced a potent reduction of Bcl-2, whereas the control ON s were ineffective. In all the conditions tested, expression of Bax remained unchanged (Fig. 11A). Densitometric analysis demonstrated an approximately 50% reduction of bcl-2/bax ratio in 5543 αᵥ antisense ON -, but not in control ON -treated cells (Fig. 11B).

### Example 8: Inhibition of cell Adhesion of Breast carcinoma MDA-MB231 cells and Determination of Apoptosis.

Antisense ON 5543 at 1 µM concentration was tested in carcinoma cells basically as described above and were found to inhibit MDA-MB231 cell adhesion to by about 40% whereas no cell adhesion decreased with the sense and mismatch control oligonucleotides. The antisense oligonucleotide was added to MBA-MB231 cells settled onto fibrinogen-coated wells at 0.01 to 1 µM concentrations. Cells were challenged for 24 hrs prior to staining with crystal violet. TUNEL staining of fragmented DNA again revealed programmed cell death of the cancer cells.

### Example 9: Inhibition of cell Adhesion of GCT23 cells.

Antisense ON 5473 at 1 µM concentration was tested in carcinoma cells basically as described above and were found to inhibit GCT23 cell adhesion by about 40% whereas cell adhesion was not decreased with the sense and inverted control oligonucleotides.

### Example 10: Inhibition of cell Adhesion by oligonucleotides of different chemical modification.

ON 5959, ON 5431, ON 5504, ON 5506 and ON 5959 were all tested as described in examples 3 and 4 and found to inhibit cell adhesion. ON 5506 gave 80 % inhibition of MBA-MB231 cell adhesion.

### Example 11: Treatment of MDA-MB231 Cells with ONs.

Incubation of MDA-MB231 cells with ON 5543 was performed in the presence of the uptake enhancer lipofectamine. Control experiments demonstrated that 5 µg/ml Lipofectamine was most effective and that up to 72 hours treatment was without toxic effect (data not shown).

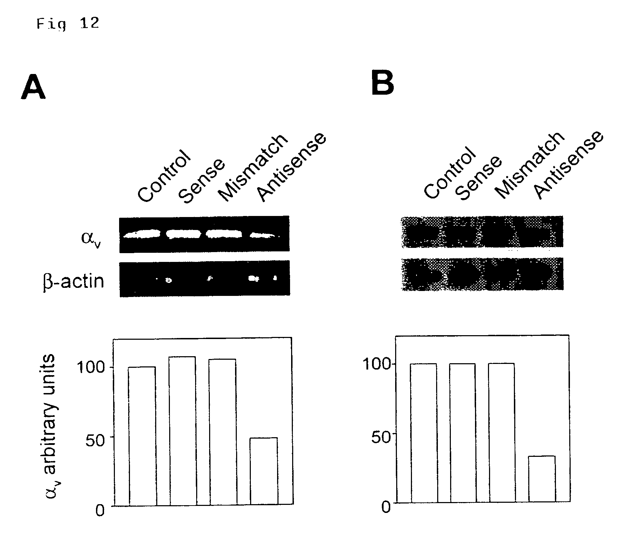

RT-PCR of cDNA and immunoblotting analysis of whole cell fraction confirmed reduction of αᵥ mRNA (55 %) and protein (65 %) in an antisense-specific manner, as demonstrated by lack of effect by the sense and mismatch 5543-ONs (Fig. 12). As reference gene, β-actin was used whose mRNA and protein expression level remained unchanged by the antisense treatment, further confirming antisense-specificity.

### Example 12: Adhesion to ECM (extracellular matrix).

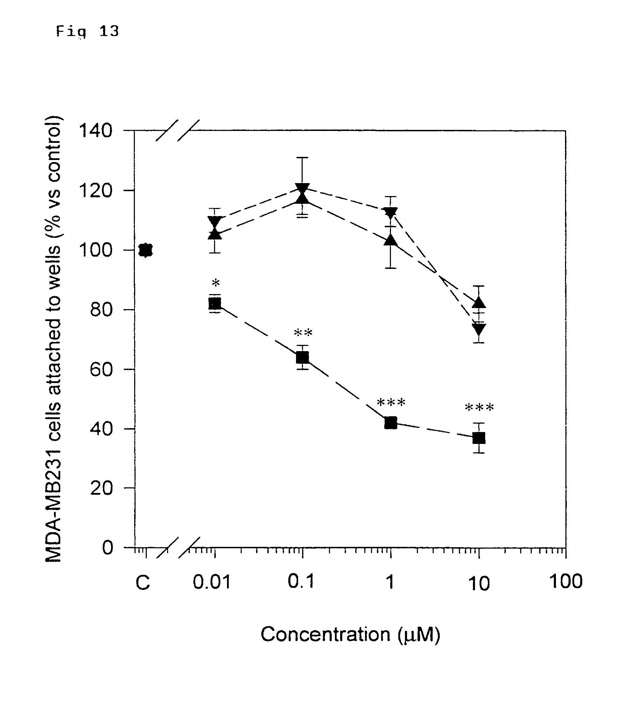

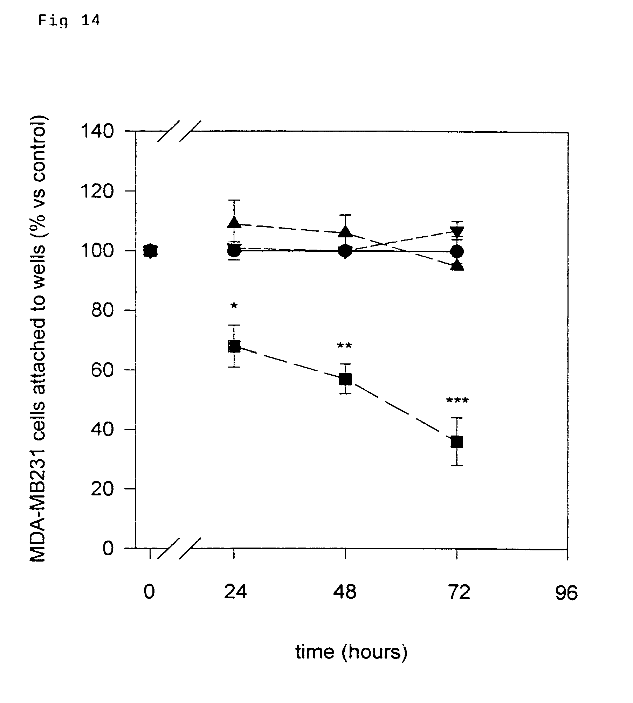

To quantitate adhesion to ECM, MDA-MB231 cells suspended in FCS-containing growth medium, were plated onto plastic wells in the presence of the αᵥ antisense ON 5543 and control ONs where the adhesion substrate was assumed to originate from the serum proteins. We observed dose-dependent inhibition of cell adhesion in cultures treated with the antisense ON 5543 with the plateau (58 % decrease) reached at 1 µM (Fig. 13). Inhibition of adhesion was time-dependent. Fig. 14 shows maximal effect (64 % decrease) on a 72 hours incubation time. All control ONs were without effect (Fig. 13 and 14.)

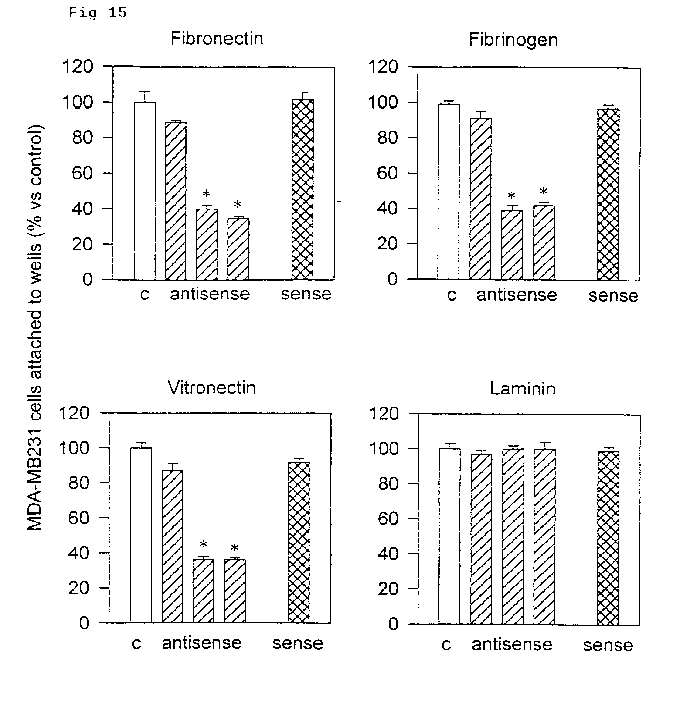

The question was addressed whether the effect of the antisense 5543-ON was ECM substrate-specific. MDA-MB231 cells were plated onto vitronectin-, fibrinogen-, fibronectin- and laminin-coated plastic. Adhesion to vitronectin, fibrinogen and fibronectin was inhibited significantly in the presence of increasing (0.1 to 1 µM) ON 5543concentrations (max. descrease 64 %, 59 % and 65 % for fibronectin, fibrinogen and vitronectin, respectively). In contrast, adhesion to laminin was unaffected. ON5044 (1 µM) used as a control was inactive (Fig. 15).

### ECM proteins and preparation and coating of glass coverslips.

13 mm glas cover slips were cleaned by 70 % ethanol washing. The cover slips were coated with 250 µl of protein solution (10 µg/ml in sterile PBS) in a 24 well tissue-culture plate at 4°C overnight. Residual protein binding sites were blocked by saturating with 1 % BSA in PBS for 1 hour at 37°C. Cover slips were then washed three times in PBS and once in DMEM. The solutions were then replaced with 60 % methanol, and the wells were further incubated for 1 h at 4°C. Methanol was removed by aspiration, and the wells were incubated with 100 µl of a buffer containing 50 mM Tris-HCl (pH 7.8), 110 mM NaCl, 5 mM CaCl₂, 1 % BSA, and 0.1 mM phenylmethylsulfonylfuoride for 30 min at room temperature. Finally, wells were washed three times with DMEM supplemented with 0.2 % BSA and immediately used for the experiment.

### Cell adhesion assay.

MDA-MB231 cells were plated in 24-well multiwell plates and incubated with the ODNs in the presence of lipofectamine for different times. At the end of incubation, wells were rinsed with PBS (3x) to remove the non-adhering cells. Each well was then treated with 20 % methanol (10 minutes), and the cells were stained with 0.5 % crystal violet in 20 methanol for 5 minutes before rinsing with distilled water and air drying for 15 minutes. Crystal violet was then solubilised with 100 µl of 0.1 N Na citrate in 50 % ethanol and transferred to 96-well microtitre plates and absorbance, which was linearly proportional to the attached cells, read spectrophotometrically at 540 nm.

### Example 13: Apoptosis of MDA-MB231 cells.

DNA was decorated with bis-benzimide, a reagent that bins the Adenine-Thymidine regions of the nucleic acid revealing its morphological appearance a number of fragmented DNA bodies bulging out from several nuclei in the ON 5543 treated cells were identified; such fragments were very few or totally absent in control sense and mismatch ON treated cells. To confirm apoptosis with a specific method which reveals DNA fragmentation at early stage, TUNEL staining was applied. TUNEL of nucleic was found, in the majority of antisense ON 5543 treated cells (data not shown). In contrast, control, sense and mismatch ON treated cultures showed staining in only a small proportion of cells, possibly revealing the "'physiologic" rate of programmed cell death in this cell line in our culture conditions.

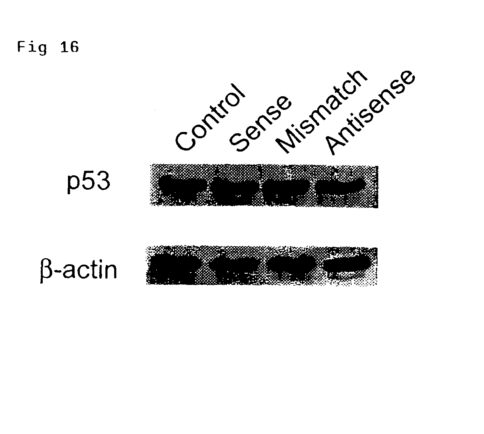

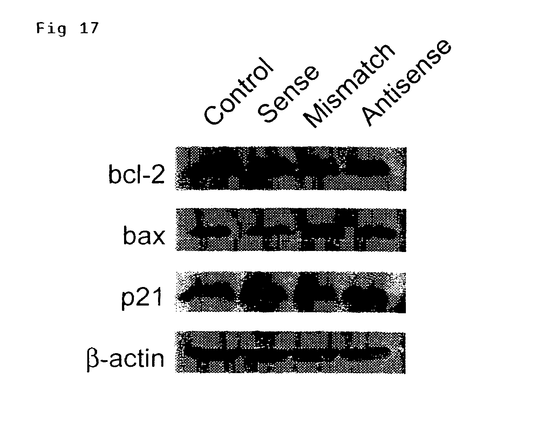

Apoptosis is known to be activated by intracellular signals which are cell type-specific and extremely heterogenous. Therefore the profile of a panel of genes potentially involved in the intracellular signal to apoptosis was investigated. MDA-MB231 cell line are known to express a mutant form of the p53 pro-apoptotic factor (22). Immunoblotting revealed p53 in our cultures. However, expression of the protein was unmodified following treatment with ON 5543 and control ONs (Fig. 16). Therefore, it was control, sense and mismatch ON-treated cultures the p53 was distributed both in the cytoplasm and in the nuclei of the MDA-MB231 cells. In contrast, in the ON 5543treated cultures the p53 was almost totally translocated to the nuclei. However, despite this, the profile of a series of genes (Bcl-2, Bax, p₂₁^{WAF1/CLP1}) whose expression can be modified by the activated p53, remained unchanged by ON 5543 treatment (Fig. 17).

### REFERENCES

1. Ruoslahti, E. and B. Öbrink. 1996. *Exp. Cell Res.* 227:1-11.
2. Humphries, M.J. 1996. *Curr. Opin. Cell Biol.* 8:632-640.
3. Meredith, J.E. Jr. and Schwartz, M.A. 1997. *Trends Cell Biol.* 7:146-150.
4. Suda, T., et al. 1997. *J. Bone Min. Res.* 12:869-879.
5. Väänänen, H.K. and M.A. Horton. 1995. *J. Cell Sci.* 108:2729-1732.
6. Vaananen, H.K. 1996. Osteoclast function: Biology and Mechanisms. In *Principles of Bone Biology.* J.P. Bilezikian, L.G. Raisz and G.A. Rodan editors, Academic Press, London. 103-113.
7. Ross, F. P., et al. 1993. *J. Biol. Chem.* 268:9901-9907.
8. Davies, J., et al. 1989. *J. Cell Biol.* 109:1817-1826.
9. Nesbitt, S., et al. 1993. *J. Biol. Chem.* 268:16737-16745.
10. Humphries, M.J. 1990. *J. Cell Sci.* 97:585-592.
11. Yamada, K.M. 1991. J. *Biol. Chem.* 266:12809-12812.
12. Bossy, B. and L.F. Reichardt. 1990. *Biochemistry* 29:10191-10198.
13. Ho, P.T.C., and D.R. Parkinson. 1997. *Semin. Oncol.* 24:187-202.
14. Sharma, H.W. and R. Narayanan. 1995. *Bioassay* 17:1005-1063.
15. Zon, G. 1995. *Tox. Letts.* 82/83:419-424.
16. Wagner, R.W. 1995. *Nat. Med.* 1:1116-1118.
17. Stein, C.A. and Cheng, Y.C. 1993. *Science* 261:1004-1012.
18. Shakin, S.H. and S.A. Liebhaber. 1986. J. *Biol. Chem.* 261:16018-16025.
19. Uhlmann, E. and A. Peyman. 1990. *Chem. Rev.* 90:543-584.
20. Schlingensiepen, R., et al. 1997. Antisense - from technology to therapy (lab manual and textbook) Blackwell Science Ex Libris Vol. 6.
21. Dean, N.M., et al. 1994. *J. Biol. Chem.* 269:16416-16424.
22. Bennett, C.F. *Cell Adhesion Molecules Meeting.* NHLI, London: IBC UK conferences.
23. Rojanasakul, Y. 1996. *Adv. Drug Delivery Rev.* 18:115-131.
24. Calbretta, B., et al. 1993. *Cancer Treat. Rev.* 19:69-179.
25. Peyman A. and E. Uhlmann. 1996. *Biol. Chem.* 377:67-70.
26. Horton, M.A., et al. 1985. *Cancer Res.* 45:5663-5669.
27. Freed, E., et al. 1989. *EMBO J.* 8:2955-2965.
28. lyer, R.P., et al. 1990. *J. Am. Chem. Soc.* 112:1253-1254.
29. Ojwand, J.O., et al. 1997. *Biochemistry* 36:6033-6045.
30. Chambers, T.J., et al. 1984. *J. Cell Sci.* 66:383-389.
31. Caselli, G.F., et al. 1997. *J. Bone Min. Res.* 12:972-981.
32. Tanaka, S., et al. 1996. *Nature* 383:528-531.
33. Prallet, R., P. et al. 1992. *J. Bone Min. Res.* 7:405-414.
34. Gavrieli, Y., et al. 1992. *J. Cell Biol.* 119:493-501.
35. Altschul, S.F., et al. 1990. *J. Mol. Biol.* 215:403-410.
37. Suzuki, S., et al. 1986. *Proc. Natl. Acad. Sci. USA* 83:8614-8618.
37. Nip, J., et al. 1995. *J. Clin. Invest.* 95:2095-2103.
38. Horton, M.A. 1997. *Int. J. Biochem. Cell Biol.* 29:721-725.
39. Rodan, S.B. and Rodan, G.A. 1997. *J. Endocrinol.* 154:S47-S56.
40. Flanagan, W.M., 1996. *Nature Biotech.* 14:1139-1145.
41. Wagner, R.W. 1994. *Nature,* 372:333-335.
42. Khaled, Z., et ak, 1989. *Nuc. Acid Res.* 24:737-645.
43. Stein, C.A. 1995. *Nature Med.* 1:1119-1121.
44. Laitala, T. and H.K. Väänänen. 1994. *J. Clin. Invest.* 93:2311-2318.
45. Laitala-Leinonen, et al. 1996. *Mol. Biol.* Cell 7:129-142.
46. Udagawa, N., et al. 1996. *Bone,* 18:511-516.
47. Inui, T., O. et al. 1997. *J. Biol. Chem.* 272:8109-8112.
48. Lakkakorpi, P.T., et al. 1991. *J. Cell Biol.* 115:1179-1186.
49. Hale, A.J., et al. 1996. *Eur. J. Biochem.* 236:1-26.
50. White, E. 1996. *Genes Develop.* 10:1-15.
51. Brooks, P.C., et al. 1994. *Science* 264:569-571.
52. Brooks, P.C., et al. 1994. *Cell* 179:1157-1164.
53. Harper, J.W., et al. 1993. *Cell* 75:805-816.
54. Strömblad, S., et al. 1996. *J. Clin. Invest.* 98:426-433.
55. Deng, C., et al. 1995. *Cell* 82:675-684.
56. McDonald, E.R. et al. 1996. *Cancer Res.* 56:2250-2255.
57. Boudreau, N., et al. 1996. *Proc. Natl. Acad. Sci. USA.* 93:3509-3513.
58. Wu, R.-C. and Schöntal, A.H. 1997. *J. Biol. Chem.* 271:29091-29098.
59. Mundy. G.R. Horm. Metabl. Res., 29: 120-127, 1997.
60. Harvey. H.A. Cancer, 80: 1646-1651, 1997.
61. Clezardin, P. Cell Mol Life Sci. 54: 541-548, 1998.
62. Hynes, R.O. Cell 48: 549-554, 1987.
63. Green, L.J., et al. lnt. J. Biochem. Cell Biol., 30: 179-184, 1998.
64. Hynes, R.O. Cell, 69: 11-25, 1992.
65. Meredith, J.E., et al. Endocrine Rev. 17: 207-220, 1996.
66. Meyer, T., et al. Br.J. Cancer 77: 530-536, 1998.
67. Marshall, J.F., et al. Cancer Biol. 7: 129-138, 1996.
68. Marshall, J.F., et al. Int. J. Cancer, 49: 924-931, 1991.
69. Gehlsen, K.R., et al. Clin. Exp. Metastasis, 10: 111-120, 1992.
70. Fattman, C.L., et al. Cancer Lett. 130: 103-113, 1998.
71. Horton, M.A. lnt. J. Biochem. Cell Biol. 29: 721-725, 1997.
72. Duong, L.T., Rodan, G.A. Front. Biosci. 3: D757-D768, 1998.
73. Stein, C.A. Antisense Nucleic Acid Drug. dev. 7201-209, 1997.
74. Brooks, P.C., et al. Cell, 79: 1157-1164, 1994.
75. Strömblad, S., et al. J. Clin. Invest. 98: 426-433, 1996.
76. Brooks, P.C., et al. J. Clin. Invest. 96: 1815-1822, 1995.
77. Villanova, I., et al. J. Bone Min. Res., submitted.
78. Hale, A.J., et al. Eur. J. Biochem. 236: 1-26, 1996.
79. Levkau, B., et al. Mol. Cell, 1: 553-563, 1998.
80. Carron, C.P., et a!. Cancer Res. 58: 1930-1935, 1998.
81. Horton, M. lnt. J. Exp. Pathol. 71:741-759, 1990.

### FIGURE LEGENDS

Fig. 1 - The ON 5543 ("5543") αᵥ sites and sequence and the rabbit αᵥ start site sequence.
   (A) The human αᵥ nucleotide site sequence is shown with the ON 5543 αᵥ included below the sequence. All sequence information is illustrated in their 5' to 3' direction with the sequence positions labeled as shown in the EMBL database. The black arrows represent the oligonucleotide primer site used in the RT-PCR cloning of the rabbit αᵥ translational start site.
   (B) Control PCR reaction showing the products when using the αᵥ primers.
   (C) RT-PCR of the rabbit αᵥ start site from a spleen and bone marrow total RNA pool, as compared to a positive control human αᵥ cDNA. Each sample was reamplified using nested primers (hence the double products in each line).
   (D) The rabbit start sequence is shown with the 5543 αᵥ ON sequence capitalized and double underlined. One base is different between the rabbit and human αᵥ cDNAs, with a T replacing a C at position 83 (due to primer degeneracy with a Y at this position).
Fig. 2 - FITC-5543 αᵥ uptake in rabbit osteoclasts.
   *Ex vivo* isolated rabbit osteoclasts were settled onto serum-coated glass and incubated with FITC labeled αᵥ 5543 antisense ON at 1 µM for 24 hours.
   (A) Top view of a multinucleated osteoclast showing distribution of FITC-5543 antisense ON to granular structures within the cytoplasm; note that nuclei are negative.
   (B) Side view of osteoclasts, taken through plane of section indicated by arrow in (A), showing granular distribution of antisense ON and absence in nuclei (glass upon which osteoclast is adherent is shown by the dotted line). Similar results were observed with osteoclasts on dentine (not shown). Magnification: X 2000.
Fig. 3 - The αᵥ antisense ON s reduce αᵥ protein expression.
   Rabbit bone marrow adherent cell fraction was cultured for 10 days with 10 nM 1,25-dihydroxy vitamin D₃ in order to differentiate a large number of TRAP-positive osteoclasts. Cultures were then treated with 1 µM αᵥ antisense and control ON s for 24 hours, lysed, subjected to SDS-PAGE (30 µg cell protein) and immunoblotted with antibodies recognizing αᵥ and β₃ integrin subunits, and actin. Primary antibodies were diluted 1:250. Secondary antibodies were diluted 1:5000. αᵥ protein level from the above gel was quantified by densitometric analysis, normalized versus actin, and expressed in arbitrary units as percent with respect to control.
Fig. 4 - The 5543 αᵥ antisense ON inhibits osteoclast adhesion and resorption.
   The αᵥ ON was added to rabbit osteoclasts settled onto serum-coated glass (A) or dentine chips (B) at increasing concentrations (5x10⁻¹³ to 100 µM). Cells were challenged for 24 hours prior to TRAP and /or pit staining. n= 8; ±S.E.M. O adhesion to serum-coated glass. ● adhesion to dentine chips. □ resorption. Δ nil. *, p<0.05; **, p<0.01; ***, p<0.001; ****, p<0.0005.
Fig. 5 - The 5543 αᵥ antisense ON binding to dentine.
   (A) FITC-linked αᵥ antisense ON binding to dentine at increasing concentrations was assessed with time from 30 minutes to 24 hours.
   (B) Represents the residual fluorescence within the media at the start (0 hours) and end point (24 hours). n=6; ±S. E. M. raw, arbitrary fluorescence units plotted versus time. ■ nil. □ 0.2 µM. ● 2 µM. O 20 µM.
Fig. 6 - Comparison of 5543 αᵥ antisense and control ONs on rabbit osteoclast function.
   Studies were performed using the αᵥ antisense ON and three relevant specificity controls. Antisense, mismatch, inverted, and sense ONs were incubated with *ex vivo* osteoclasts (0.2 to 20 µM) for 24 hours.
   (A) Adhesion to serum-coated glass (% adhesion to glass vs. Concentration ON [µM]).
   (B) Adhesion to dentine discs. (C) Resorption. ● antisense. ○ mismatch. ■ inverted. □ sense. Δ nil. n=3; ±S.E.M. *, p<0.001 (% adhesion to bone vs. Concentration of ON [µM]).
   (C) % resorption vs concentration of oligonucleotide [µM]
Fig. 7 - The 5543 αᵥ antisense ON effect on osteoclasts differentiated *in vitro.*
   The rabbit bone marrow adherent cell fraction was cultured for 10 days with 10 nM 1,25-dihydroxy vitamin D₃ and 1 mM of the indicated ONs. At the end of incubation, cells were stained for TRAP enzyme and the number of TRAP-positive (A) mononuclear (MNC) and (B) multinucleated (PNC) cells were counted and expressed as a percent rate versus untreated cultures. a'sense, antisense ON. sense, sense ON. mism, mismatch ON n=3; ±S.E.M. ***, *p*<10⁻⁶
Fig. 8 - Dose response effects of the 5543 αᵥ antisense ON on ex *vivo* rabbit osteoclasts adhered to different extracellular matrix proteins.
   Parallel studies were performed with the sense control ON (here shown at 20µM concentration only). Cultures were treated for 24 hours post attachment onto the indicated substrate protein-coated glass. (A) FBS, Fetal Bovine Serum. (B) Vn, Vitronectin. (C) Fb, Fibrinogen. (D) Fn, Fibronectin. (E) Fibrillar Collagen. (F) Table showing comparison between the effect of the αvβ₃ neutralizing antibody 23C6 and the 5543 αᵥ ON. In parenthesis the percent variation vs. nil is shown a/s, antisense ON (from left 0 µM), 0.2 µM, 2 µM, 20 µM). s, sense ON (20 µM). n=3; ±S.E.M. *, *p*<0.05; **, p<0.01; ***, p<0.001.
Fig. 9 - The 5543 αᵥ antisense ON effect on osteoclast morphology and apoptosis. *Ex vivo* rabbit osteoclasts were incubated with the indicated ONs for 24 hours.
   (A-D) Phase contrast micrographs. Arrow indicates a retracted osteoclast. Magnification X 1400.
   (E-H) Bisbenzimide nuclear staining. Arrow indicates apoptotic bodies. Magnification X 1400. (I-L) TUNEL staining of fragmented DNA. Magnification X 700. A,E,I: control (c). B,F,J: sense ON (s). C,G,K: mismatch ON (m). D,H,L: antisense ON (a/s).
Fig. 10 - The 5543 aᵥ antisense ON stimulates p21^{WAF1/CIP1} immunoreactivity. Untreated ex *vivo* rabbit osteoclasts (A) and osteoclasts incubated for 48 hours with 1 mM of (B) sense, (C) mismatch and (D, E) antisense ONs, were fixed and subjected to immunofluorescence staining with an antibody recognizing the p21^{WAF1/CIP1} protein. Arrows indicate p21^{WAF1/CIP1}-positive osteoclasts. Magnification X 1000. (F) The number of p21^{WAF1/CIP1} immunoreactive mononuclear cells and the total number of mononuclear cells present in at least 5 random microscopic field (objective 20X) were counted in control and in antisense (a'sense)-, sense- and mismatch (mism)-treated cultures, and the percent of positive versus total cells calculated. n=3±S.E.M. ***p<0.0001.
Fig. 11 - Expression of the bcl-2 and bax genes.
   Cells of the osteoclast lineage were obtained as described in Fig. 3 and treated with 1 mM aᵥ antisense and control ONs for 48 hours. Cells were then lysated and subjected to analysis of bcl-2 and bax protein levels by Western blotting as described in Methods. The bcl-2 and bax protein levels were then quantified by densitometry upon normalization versus actin expression. The bars represent the relative ratio as measured by densitometric analysis and not the absolute molar ratio. c: control. s: sense ON. m: mismatch ON. a/s: antisense ON.
Fig. 12 - Analysis of αᵥ integrin expression in MDA-MB231 cells.
   90 % confluent MDA-MB231 cell monolayers were treated for 72 hours with 1 µM ON5543.
   (A) RT-PCR. 0.1 µg RNA was reverse transcribed and PCR reactions were run with the αᵥ and the β-actin primer pairs. For semiquantitative assay, densitometric analysis was carried out, and each value was coverted as present of αᵥ/β-actin ration of ON5543-treated versus control cells. The experiment was repeated three times with similar results.
   (B) Immunoblotting. 15 µg protein were subjected to SDS-Page (7.5 %) acrylamide gel) under non-reducing condition and transferred to a Hybond nitrocellulose membrane. The filter was incubated with the primary antibodies (diluted 1:200) overnight at 4°C, and with the HRP-conjugated secondary antibodies (diluted 1:5000) for 1 h at 37°C. Bands were detected by ECL and analysed by densitomety. Results are expressed as percent of the αᵥ/β-actin ratio of ON5543-treated versus control cells. Similar results were observed in three independent experiments.
Fig. 13 - Concentration-dependent inhibition of MDA-MB231 cell adhesion to substrate
   MDA-MD231 cells cultured in DMEM with ON 5543 at the concentrations indicated on the abscissa. Incubation was proceeded for 72 hours, then the cultures were fixed, extensively washed to remove floating cells, and nuclei were stained with crystal violet. The staining, which was assumed to be proportional to the number of adherent cells, was then solubilized and colour intensity was measured by spectrophotometry as described in the examples. Data are expressed as percent versus control. Mean ± S.E.M. n = 3, p<0.003. 1 = ON5543; 2 = ON5044; 3 = ON5045.
Fig. 14 - Time dependent inhibition of MDA-MB231 cell adhesion.
   MDA-MB231 cells were cultured in DMEM containing 1 µM ON5543. Incubation was proceeded for the times indicated on the abscissa, then the cells were fixed, and adhesion was measured as described in Fig. 13. Data are the average ± S.E.M. of percent versus control. n = 3, p<0.003. 1 = Control; 2 = ON5543; 3 = ON5044; 4 = ON5045.
Fig. 15 - Effect of αᵥ antisense ON5543 on adhesion of MDA-MB231 cells to ECM substrates (% vs control).
   MDA-MB231 cells were plated in fibronectin- (I), fibrinogen- (II), vitronectin- (III) and laminin-coated wells (IV) and incubated for 72 hours in serum-free DMEM containing 0.2 % BSA with 0.01, 0.1 and µM ON5543 or 1 µM ON5044. Cells were then fixed and adhesion was measured as described in Fig. 13. Data are expressed as percent versus control. Mean ± S.E.M. n = 3. p<0.0001; 1 = control; 2 = ON5543 (0.01 µM); 3 = ON5543 (0.1 µM); 4 = ON5543 (1 µM); 5 = ON5044 (1 µM).
Fig. 16 - Analysis of p53 expression and distribution.
   90 % confluent MDA-MB231 cell monolayers were treated for 72 hours with 1 µM ON5543.
   (A) Immublotting.
      40 µg protein were subjected to SDS-PAGE (15 % acrylamide gel) under non-reducing condition and transferred to a Hybond nitrocellulose membrane. Membranes were incubated with the primary antibodies (diluted: 1:200) overnight at 4°C, and with the secondary HRP-conjugated secondary antibodies (diluted: 1:5000) for 1 h at 37°C. Band were detected by ECL.
   (B) Immunofluorescence.
      Cells were fixed, immunostained with anti-p53 antibody and observed by conventional epifluorescence microscopy. (a) Control without ON; (b) ON5044; (c) ON5045; (d) ON5543. Magnification 300X. Note cytosolic and nuclear distribution of p53 in (a), (b) and (c), and nuclear staining only in (d).
Fig. 17 - Immunoblotting analysis of genes potentially involved in apoptosis.
   90 % confluent MDA-MB231 cell monolayers were treated for 72 hours with 1 µM ON5543. 80 µg protein were subjected to SDS-PAGE (15 % acrylamide gel) under non-reducing condition and transferred to a Hybond nitrocellulose membranes. The filters were then probed with anti-bcl2, -bax, -p21,a nd β-actin (internal control) antibodies. Membranes were incubated with the primary antibodi3es (diluted: 1:2000) overnight at 4°C, and with the secondary HRP-conjugated secondary antibodies (diluted 1:5000) for 1 h at 37°C. Bands were detected by ECL.

## Claims

1. An antisense oligonucleotide or a derivative thereof which has or comprises one of the sequences SEQ ID NO. 4, SEQ ID NO. 5, SEQ ID NO. 6, SEQ ID NO. 7, SEQ ID NO. 8 or SEQ ID NO. 12 and which
a) has a sequence that corresponds to a part of a nucleic acid which encodes integrin αᵥ and
b) induces apoptosis in a cell that contains integrin αᵥ mRNA, when brought into that cell, wherein
SEQ ID NO. 4 is 3'- GAAGCCGCTACCGAAAAGGC -5'
SEQ ID NO. 5 is 3'- CGCGTGAAGCCGCTACCG -5'
SEQ ID NO. 6 is 3'- GCTACCGAAAAGGCGGCG -5'
SEQ ID NO. 7 is 3'- GCTGCCGAGAGAGCAACG -5'
SEQ ID NO. 8 is 3'- GCGGAAGTTGGATCTGC -5' and
SEQ ID NO. 12 is 3'- GCGGAAGTTGGACCTGC -5'.

2. An antisense oligonucleotide or a derivative thereof as claimed in claim 1, wherein 1 - 5 nucleotides at the 3' and/or the 5' end and at least one non-terminal pyrimidine nucleoside and/or a phosphodiester bridge located at the 3' and/or the 5' end of that pyrimidine nucleoside are modified, wherein each modification is independently selected from
a) the replacement of a phosphoric diester bridge located at the 3'- and/or the 5'- end of a nucleoside by a modified phosphodiester bridge,
b) the replacement of phosphoric diester bridge located at the 3'- and/or the 5'-end of a nucleoside by a dephospho bridge,
c) the replacement of a sugar phosphate molecule from the sugarphosphate backbone by an other molecule,
d) the replacement of a β**-**D-2'-deoxyribose by a modified sugar radical,
e) the replacement of a natural nucleoside base by a modified base,
f) the conjugation of the oligonucleotide to a molecule which influences the properties of the oligonucleotide and
g) the introduction of a 3'-3' and/or a 5'-5' inversion at the 3' and/or the 5' end of the oligonucleotide.

3. A process for the preparation of an oligonucleotide as claimed in claim 1 or 2, by condensing suitably protected monomers on a solid support.

4. A method for inhibiting the expression of integrin αᵥ in a cell, wherein an antisense oligonucleotide according to claim 1 is made and hybridized to integrin αᵥ mRNA in vitro.

5. A method as claimed in claim 4 , wherein the cell is a tumor cell or an osteoclast cell.

6. A method of modulating the expression or activity of a protein which is involved in at least one of the signal transduction pathways for inducing apoptosis, wherein an antisense oligonucleotide according to claim 1 is made and brought into a cell cultured in vitro, whereupon the antisense oligonucleotide or the derivative thereof hybridizes to integrin αᵥ mRNA and thereby inhibits the expression of integrin αᵥ to certain extend.

7. The use of an antisense oligonucleotide or a derivative thereof as claimed in any of claims 1 or 2 for the preparation of a pharmaceutical composition for treating deseases which are associated with the expression or an increased expression of integrin αᵥ.

8. A method for preparing a pharmaceutical composition, which comprises mixing of at least one antisense oligonucleotide or a derivative thereof as claimed in any of claims or 2 with a physiologically acceptable excipients and if appropriate suitable additives and/or auxiliaries.

9. A pharmaceutical composition which comprises at least one oligonucleotide as claimed in any of claims 1 or 2 and if appropriate one or more physiologically acceptable excipients and suitable additives and/or auxiliaries.

10. A pharmaceutical composition as claimed in claim 9, for the treatment and/or prevention of diseases which are associated with abnormal cell proliferation, cell migration, cell differentiation, angiogenesis, retinal neurite outgrowth, bone resorption, phagocytosis, immune response, signal transduction and the metastasis of neoplastic cells.

11. A pharmaceutical composition as claimed in one or more of claims 9 or 10, for the treatment and prevention of cancer and metastasis of cancer, the treatment and prevention of osteoporosis, the treatment of ocular diseases, chronic inflammation, psorasis, restenosis and the support of wound healing.

12. A method for identifying cells, which express or overexpress integrin αᵥ, wherein (a) an oligonucleotide or a derivative thereof as claimed in any of claims 1 or 2 is synthesized and brought into contact with a cell or a probe of a cell, and (b) detecting it is analyzed, if the antisense oligonucleotid or derivative thereof has hybridized to integrin αᵥ mRNA.

13. A test kit or a diagnostic reagent for identifying cells which express or overexpress integrin αᵥ, which comprises
a) an oligonucleotide according to claim 1 and
b) a reagent for detecting, if the oligonucleotide or derivative thereof has hybridized to integrin αᵥ mRNA.

## Patentansprüche

1. Antisense-Oligonucleotid oder ein Derivat davon, das eine der Sequenzen SEQ ID NR. 4, SEQ ID NR. 5, SEQ ID NR. 6, SEQ ID NR. 7, SEQ ID NR. 8 oder SEQ ID NR. 12 aufweist oder umfasst, und das
a) eine Sequenz aufweist, die einem Teil einer Nucleinsäure entspricht, die Integrin αᵥ codiert und
b) Apoptose in einer Integrin αᵥ-mRNA enthaltenden Zelle induziert, wenn in die Zelle gebracht, wobei
SEQ ID NR. 4 3'-GAAGCCGCTACCGAAAAGGC-5' ist,
SEQ ID NR. 5 3'-CGCGTGAAGCCGCTACCG-5' ist,
SEQ ID NR. 6 3'-GCTACCGAAAAGGCGGCG-5' ist,
SEQ ID NR. 7 3'-GCTGCCGAGAGAGCAACG-5' ist,
SEQ ID NR. 8 3'-GCGGAAGTTGGATCTGC-5' ist und
SEQ ID NR. 12 3'-GCGGAAGTTGGACCTGC-5' ist.

2. Antisense-Oligonucleotid oder ein Derivat davon nach Anspruch 1, worin 1 - 5 Nucleotide an dem 3' und/oder dem 5'-Ende und mindestens ein nicht-terminales Pyrimidin-Nucleosid und/oder eine Phosphodiesterbrücke, lokalisiert an dem 3' und/oder dem 5'-Ende des Pyrimidin-Nucleosids, modifiziert sind, wobei die Modifizierung unabhängig ausgewählt ist aus
a) dem Ersatz einer Phosphorsäurediesterbrücke, lokalisiert an dem 3' und/oder dem 5'-Ende von einem Nucleotid, durch eine modifizierte Phosphodiesterbrücke,
b) dem Ersatz einer Phosphorsäurediesterbrücke, lokalisiert an dem 3' und/oder dem 5'-Ende von einem Nucleotid, durch eine Dephospho-Brücke,
c) dem Ersatz eines Zuckerphosphatmoleküls aus dem Zuckerphosphatgerüst durch ein anderes Molekül,
d) dem Ersatz einer β-D-2'-Desoxyribose durch einen modifizierten Zuckerrest,
e) dem Ersatz einer natürlichen Nucleosidbase durch eine modifizierte Base,
f) der Konjugation des Oligonucleotids an ein Molekül, das die Eigenschaften des Oligonucleotids beeinflusst, und
g) der Einführung einer 3'-3' und/oder 5'-5' Inversion an dem 3' und/oder dem 5'-Ende von dem Oligonucleotid.

3. Verfahren zur Herstellung eines Oligonucleotids nach Anspruch 1 oder 2 durch Kondensieren geeignet geschützter Monomere an einen festen Träger.

4. Verfahren zum Inhibieren der Expression von Integrin αᵥ in einer Zelle, wobei ein Antisense-Oligonucleotid nach Anspruch 1 hergestellt und in vitro an Integrin αᵥ-mRNA hybridisiert wird.

5. Verfahren nach Anspruch 4, wobei die Zelle eine Tumorzelle oder eine Osteoklastenzelle ist.

6. Verfahren zum Modulieren der Expression oder Aktivität von einem Protein, das in mindestens einen der Signaltransduktions-Pathways einbezogen ist, zum Induzieren von Apoptose, wobei ein Antisense-Oligonucleotid nach Anspruch 1 hergestellt und in eine in vitro kultivierte Zelle gebracht wird, wonach das Antisense-Oligonucleotid oder das Derivat davon an Integrin αᵥ-mRNA hybridisiert und **dadurch** die Expression von Integrin αᵥ zu einem bestimmten Ausmaß inhibiert.

7. Verwendung von einem Antisense-Oligonucleotid oder einem Derivat davon nach einem der Ansprüche 1 oder 2 für die Herstellung einer pharmazeutischen Zubereitung zur Behandlung von Erkrankungen, die mit der Expression oder einer erhöhten Expression von Integrin αᵥ verbunden sind.

8. Verfahren zur Herstellung einer pharmazeutischen Zusammensetzung, das Vermischen von mindestens einem Antisense-Oligonucleotid oder einem Derivat davon nach einem der Ansprüche 1 oder 2 mit einem physiologisch verträglichen Exzipienten und, falls geeignet, geeigneten Additiven und/oder Hilfsmitteln umfasst.

9. Pharmazeutische Zusammensetzung, die mindestens ein Oligonucleotid nach einem der Ansprüche 1 oder 2 und, falls geeignet, einen oder mehrere physiologisch verträgliche Exzipienten und geeignete Additive und/oder Hilfsmittel umfasst.

10. Pharmazeutische Zusammensetzung nach Anspruch 9 für die Behandlung und/oder Prävention von Erkrankungen, die mit abnormaler Zellproliferation, Zellmigration, Zelldifferenzierung, Angiogenese, retinalem Neuritenwachstum, Knochenresorption, Phagozytose, Immunreaktion, Signaltransduktion und der Metastasierung von neoplastischen Zellen verbunden sind.

11. Pharmazeutische Zubereitung nach einem oder mehreren von Ansprüchen 9 oder 10, für die Behandlung und Prävention von Krebs und Metastasierung von Krebs, die Behandlung und Prävention von Osteoporose, die Behandlung von Augenerkrankungen, chronischer Entzündung, Psoriasis, Restenose und die Unterstützung von Wundheilung.

12. Verfahren zum Identifizieren von Zellen, die Integrin αᵥ exprimieren oder überexprimieren, wobei (a) ein Oligonucleotid oder ein Derivat davon nach einem der Ansprüche 1 oder 2 synthetisiert und mit einer Zelle oder einer Sonde von einer Zelle in Kontakt gebracht wird und (b) Nachweisen, dass sie analysiert ist, wenn das Antisense-Oligonucleotid oder Derivat davon an Integrin αᵥ-mRNA hybridisiert hat.

13. Test-Kit oder Diagnosereagenz zum Identifizieren von Zellen, die Integrin αᵥ exprimieren oder überexprimieren, das umfasst
a) ein Oligonucleotid nach Anspruch 1 und
b) ein Reagenz zum Nachweisen, wenn das Oligonucleotid oder Derivat davon an Integrin αᵥ-mRNA hybridisiert hat.

## Revendications

1. Oligonucléotide antisens, ou dérivé de ce dernier, qui possède ou comprend l'une des séquences SEQ ID N°: 4, SEQ ID N°: 5, SEQ ID N°: 6, SEQ ID N°: 7, SEQ ID N°: 8 ou SEQ ID N°: 12, et qui
a) possède une séquence qui correspond à une partie de l'acide nucléique qui code pour l'intégrine αᵥ, et
b) induit une apoptose dans une cellule qui contient l'ARNm de l'intégrine αᵥ quand il est introduit dans cette cellule, où
SEQ ID N°: 4 est 3'-GAAGCCGCTACCGAAAAGGC-5'
SEQ ID N°: 5 est 3'-CGCGTGAAGCCGCTACCG-5'
SEQ ID N°: 6 est 3'-GCTACCGAAAAGGCGGCG-5'
SEQ ID N°: 7 est 3'-GCTGCCGAGAGAGCAACG-5'
SEQ ID N°: 8 est 3'-GCGGAAGTTGGATCTGC-5' et
SEQ ID N°: 12 est 3'-GCGGAAGTTGGACCTGC-5'

2. Oligonucléotide antisens ou dérivé de ce dernier selon la revendication 1, dans lequel 1-5 nucléotides à l'extrémité 3' et/ou à l'extrémité 5', et au moins un pyrimidine-nucléoside non terminal et/ou un pont phosphodiester situé à l'extrémité 3' et/ou à l'extrémité 5' de ce pyrimidine-nucléoside, sont modifiés, où chaque modification est d'une manière indépendante choisie parmi :
a) le remplacement du pont diester d'acide phosphorique situé à l'extrémité 3' et/ou à l'extrémité 5' d'un nucléoside par un pont phosphodiester modifié,
b) le remplacement du pont diester d'acide phosphorique situé à l'extrémité 3' et/ou à l'extrémité 5' d'un nucléoside par un pont déphospho,
c) le remplacement d'une molécule de sucre-phosphate, provenant du squelette sucre-phosphate, par une autre molécule,
d) le remplacement d'un β-D-2'-désoxyribose par un radical sucre modifié,
e) le remplacement d'une base nucléosidique naturelle par une base modifiée,
f) la conjugaison de l'oligonucléotide à une molécule qui influe sur les propriétés de l'oligonucléotide, et
g) l'introduction d'une inversion 3'-3' et/ou d'une inversion 5'-5', à l'extrémité 3' et/ou à l'extrémité 5' de l'oligonucléotide.

3. Procédé de préparation d'un oligonucléotide selon la revendication 1 ou 2, par condensation, sur un support solide, de monomères convenablement protégés.

4. Procédé pour inhiber l'expression de l'intégrine αᵥ dans une cellule, dans lequel un oligonucléotide antisens selon la revendication 1 est fabriqué et hybridé in vitro à l'ARNm de l'intégrine αᵥ.

5. Procédé selon la revendication 4, dans lequel la cellule est une cellule tumorale ou une cellule ostéoclaste.

6. Procédé de modulation de l'expression ou de l'activité d'une protéine qui est impliquée dans au moins l'une des voies de transduction de signaux dans le but d'induire une apoptose, dans lequel un oligonucléotide antisens selon la revendication 1 est fabriqué et introduit dans une cellule cultivée in vitro, ce après quoi l'oligonucléotide antisens ou son dérivé va s'hybrider à l'ARNm de l'intégrine αᵥ et de ce fait inhiber dans une certaine mesure l'expression de l'intégrine αᵥ.

7. Utilisation d'un oligonucléotide antisens ou d'un dérivé de ce dernier selon l'une quelconque des revendications 1 ou 2 pour préparer une composition pharmaceutique destinée au traitement de maladies qui sont associées à l'expression ou à une expression accrue de l'intégrine αᵥ.

8. Procédé de préparation d'une composition pharmaceutique, qui comprend le mélange d'au moins un oligonucléotide antisens ou d'un dérivé de ce dernier selon l'une quelconque des revendications 1 ou 2 avec des excipients acceptables d'un point de vue physiologique et, si cela est approprié, avec des additifs et/ou auxiliaires appropriés.

9. Composition pharmaceutique qui comprend au moins un oligonucléotide selon l'une quelconque des revendications 1 ou 2 et, si cela est approprié, un ou plusieurs excipients acceptables d'un point de vue physiologique et des additifs et/ou auxiliaires appropriés.

10. Composition pharmaceutique selon la revendication 9, pour le traitement et/ou la prévention de maladies qui sont associées à une prolifération cellulaire anormale, à une migration de cellules, à une différenciation cellulaire, à une angiogenèse, à une excroissance des neurites de la rétine, à une résorption osseuse, à une phagocytose, à une réponse immune, à une transduction de signaux et à la métastase de cellules néoplasiques.

11. Composition pharmaceutique selon l'une ou plusieurs des revendications 9 ou 10, pour le traitement et la prévention du cancer et de la métastase du cancer, pour le traitement et la prévention de l'ostéoporose, pour le traitement des maladies oculaires, de l'inflammation chronique, du psoriasis, de la resténose, et pour faciliter la cicatrisation.

12. Procédé pour identifier des cellules qui expriment ou surexpriment l'intégrine (αᵥ, dans lequel (a) un oligonucléotide ou un dérivé de ce dernier selon l'une quelconque des revendications 1 ou 2 est synthétisé et mis en contact avec une cellule ou une sonde d'une cellule, et (b) on détecte si l'oligonucléotide antisens ou son dérivé s'est hybridé à l'ARNm de l'intégrine αᵥ.

13. Trousse d'essai ou réactif de diagnostic pour identifier des cellules qui expriment ou surexpriment l'intégrine αᵥ, qui comprend
a) un oligonucléotide selon la revendication 1, et
b) un réactif pour détecter si l'oligonucléotide ou son dérivé s'est hybridé à l'ARNm de l'intégrine αᵥ.


### SEQUENCE LISTING

<110> Hoechst Marion Roussel Deutschland GmbH
<120> Antisense Oligonucleotides for the Inhibition of Integrin αᵥ-Subunit Expression
<130> 1998/L017
<140> PCT/EP99/02286
   <141> 1999-04-01
<150> EP 98106989.1
   <151> 1998-04-17
<160> 14
<170> PatentIn Ver. 2.1
<210> 1
   <211> 540
   <212> DNA
   <213> Homo sapiens
<400> 1
   ggctaccgct cccggcttgg cgtcccgcgc gcacttcggc gatggctttt ccgccgcggc 60
   gacggctgcg cctcggtccc cgcggcctcc cgcttcttct ctcgggactc ctgctacctc 120
   tgtgccgcgc cttcaaccta gacgtggaca gtcctgccga gtactctggc cccgagggaa 180
   gttacttcgg cttcgccgtg gatttcttcg tgcccagcgc gtcttcccgg atgtttcttc 240
   ggctaccgct cccggcttgg cgtcccgcgc gcacttcggc gatggctttt ccgccgcggc 300
   ccgatggcga gggccgaacc gcagggcgcg cgtgaagccg ctaccgaaaa ggcggcgccg 360
   ccgatggcga gggccgaacc gcagggcgcg cgtgaagccg ctaccgaaaa ggcggcgccg 420
   ccgatggcga gggccgaacc gcagggcgcg cgtgaagccg ctaccgaaaa ggcggcgccg 480
   ccgatggcga gggccgaacc gcagggcgcg cgtgaagccg ctaccgaaaa ggcggcgccg 540
<210> 2
   <211> 24
   <212> DNA
   <213> Homo sapiens
<400> 2
   cggcgatggc ttttccgccg cggc 24
<210> 3
   <211> 26
   <212> DNA
   <213> Homo sapiens
<400> 3
   gtgccgcgcc ttcaacctag acgtgg 26
<210> 4
   <211> 20
   <212> DNA
   <213> Artificial Sequence
<220>
   <223> Description of Artificial Sequence: antisense
<400> 4
   cggaaaagcc atcgccgaag 20
<210> 5
   <211> 18
   <212> DNA
   <213> Artificial Sequence
<220>
   <223> Description of Artificial Sequence: antisense
<400> 5
   gccatcgccg aagtgcgc 18
<210> 6
   <211> 18
   <212> DNA
   <213> Artificial Sequence
<220>
   <223> Description of Artificial Sequence: antisense
<400> 6
   gcggcggaaa agccatcg 18
<210> 7
   <211> 18
   <212> DNA
   <213> Artificial Sequence
<220>
   <223> Description of Artificial Sequence: antisense
<400> 7
   gcaacgagag agccgtcg 18
<210> 8
   <211> 17
   <212> DNA
   <213> Artificial Sequence
<220>
   <223> Description of Artificial Sequence: antisense
<400> 8
   cgtctaggtt gaaggcg 17
<210> 9
   <211> 18
   <212> DNA
   <213> Artificial Sequence
<220>
   <223> Description of Artificial Sequence: antisense
<400> 9
   gcccgggagc agccatcg 18
<210> 10
   <211> 18
   <212> DNA
   <213> Artificial Sequence
<220>
   <223> Description of Artificial Sequence: antisense
<400> 10
   gctaccgaaa aggcggcg 18
<210> 11
   <211> 18
   <212> DNA
   <213> Artificial Sequence
<220>
   <223> Description of Artificial Sequence: antisense
<400> 11
   cgatggcttt tccgccgc 18
<210> 12
   <211> 17
   <212> DNA
   <213> Artificial Sequence
<220>
   <223> Description of Artificial Sequence: antisense
<400> 12
   cgtccaggtt gaaggcg 17
<210> 13
   <211> 17
   <212> DNA
   <213> Artificial Sequence
<220>
   <223> Description of Artificial Sequence: antisense
<400> 13
   cgtccaggtt gaaggcg 17
<210> 14
   <211> 17
   <212> DNA
   <213> Artificial Sequence
<220>
   <223> Description of Artificial Sequence: antisense
<400> 14
   gcaggtccaa cttccgc 17